(19) Europäisches Patentamt

European Patent Office

Office européen des brevets

(11) **EP 1 008 658 A1**

(12) **EUROPEAN PATENT APPLICATION**
published in accordance with Art. 158(3) EPC

(43) Date of publication:
**14.06.2000 Bulletin 2000/24**

(51) Int. Cl.⁷: **C12Q 1/68**, C12N 15/10

(21) Application number: **99901147.1**

(86) International application number:
**PCT/JP99/00252**

(22) Date of filing: **22.01.1999**

(87) International publication number:
**WO 99/60158 (25.11.1999 Gazette 1999/47)**

(84) Designated Contracting States:
**DE FR GB**

(30) Priority: **19.05.1998 JP 15384098**

(71) Applicant:
**Laboratory of Molecular Biophotonics
Hamakita-shi, Shizuoka 434-8555 (JP)**

(72) Inventors:
• **ABE, Satoshi,
Lab. of Molecular Biophotonics
Hamakita-shi, Shizuoka 434-8555 (JP)**

• **TOYONAGA, Shuji,
Lab. of Molecular Biophotonics
Hamakita-shi, Shizuoka 434-8555 (JP)**

(74) Representative:
**Grünecker, Kinkeldey,
Stockmair & Schwanhäusser
Anwaltssozietät
Maximilianstrasse 58
80538 München (DE)**

(54) **SOLID PHASE FOR DETECTING NUCLEIC ACID AND METHOD FOR DETECTING NUCLEIC ACID**

(57) A solid phase for detecting a target nucleic acid and a method for detecting a target nucleic acid with the use of the same. The fundamental structure of the above solid phase comprises a fluorochrome bonded, via a linker, to one end of an oligonucleotide containing a base sequence complementary to the target nucleic acid and a probe having a solid-phase binding site bonded, via the solid-phase bond, to the other end. The above detection method comprises measuring the degree of polarization of the fluorescence generated from the probe and thus detecting the target nucleic acid.

**Fig.2**

EP 1 008 658 A1

## Description

### TECHNICAL FIELD

This invention relates to a solid phase system for the detection of a target nucleic acid, a method for the detection, the quantification, or the detection of two-dimensional distribution of a target nucleic acid using the solid phase system.

### BACKGROUND ART

Detection of nucleic acids having specified base sequences is in wide use for the diagnosis of a variety of gene disorders as well as for the identification of pathogenic microorganisms. In the detection of such a nucleic acid having a specified base sequence, frequently used are probes where oligonucleotides comprising base sequences complementary to those to be detected are labeled with radioisotopes, enzymes, fluorescent dyes, or the like. The presence of the nucleic acid having a specified base sequence is detected by recognizing the bonding between such a probe and the target nucleic acid. Such bonding between the probe and the target nucleic acid is usually detected after the probe bound to the target nucleic acid has been separated from unbound one by some kind of means, which thus brings complexity.

It is known that the degree of polarization of the fluorescence emitted by a fluorescent dye labeled at a nucleic acid varies when the fluorescent dye binds to another nucleic acid having a base sequence complementary to said nucleic acid. There have been reports of methods to detect nucleic acids having specified base sequences which relay on this technique. (Publications of Japanese Unexamined Application Hei 2-75958 and Hei 5-123196 and Published Japanese Translation of PCT International Publication Hei 8-505285.) These methods do not physically separate probes bound to target nucleic acids from unbound ones, and can detect bonding between the probes and the target nucleic acids, namely the nucleic acids having specified base sequences, through variation in the degree of polarization of the fluorescence emitted by the probes; and they are very convenient.

However, the aforementioned methods measure the degree of polarization of the fluorescence emitted by the probes in reaction solutions. Thus they are advantageous to detecting the presence of one kind of base sequence contained in a sample but find the difficulty in detecting the presence of two or more kinds of base sequence at the same time, which presents a problem.

Further, since in the method to be carried out in solution the probe and the sample are both present in the solution, there is a problem that it is difficult to separately recover the sample and the probe and to reuse them.

There is also another problem with the method to be carried out in solution that it is difficult to apply the method to one for detecting the two-dimensional distribution of a nucleic acid to be detected: for example, the histochemical detection of the nucleic acid having a specified sequence existing in certain part of a biological tissue.

### DISCLOSURE OF INVENTION

An object of this invention is to solve the aforementioned problems and to provide a solid phase system for the detection of a nucleic acid to conveniently and quickly detect one or plural target nucleic acids existing in solution, as well as a method for the detection of a target nucleic acid using the solid phase system. Furthermore, another object is to provide a method for the quantification, or the detection of two-dimensional distribution of a target nucleic acid using the solid phase system.

### BRIEF DESCRIPTION OF DRAWINGS

Fig. 1A is an illustration showing an embodiment of the solid phase system according to this invention.
Fig. 1B is an illustration showing another embodiment of the solid phase system according to the invention.
Fig. 1C is an illustration showing a further embodiment of the solid phase system according to the invention.
Fig. 1D is an illustration showing a still further embodiment of the solid phase system according to the invention.
Fig. 1E is an illustration showing another embodiment of the solid phase system according to the invention.
Fig. 1F is an illustration showing another embodiment of the solid phase system according to the invention.
Fig. 2 is an illustration showing the method according to the invention using a solid phase system according to the invention.
Fig. 3A is an illustration showing an application example utilizing the method according to the invention: the example where the kind of probe is different in each region.

Fig. 3B is an illustration showing another application example utilizing the method according to the invention: the example where the density of probe is different in each region.

Fig. 3C is an illustration showing a further application example utilizing the method according to the invention: the example where the kind and the density of probe are both uniform.

Fig. 4 is photographs showing the fluorescence images taken using the solid phase system according to the invention (mode-locked titanium sapphire laser excitation). From the left, the case where no target nucleic acid was present, the case where the target nucleic acid of polythymine sequence was added, and the case where the target nucleic acid of polyadenine sequence was additionally added are indicated. Here, the upper halves are probe regions having polyadenine sequences and the lower halves are probe regions having polythymine sequences.

Fig. 5 is photographs showing the images of the degree of polarization taken using the solid phase system according to the invention (mode-locked titanium sapphire laser excitation). From the left, the case where no target nucleic acid was present, the case where the target nucleic acid of polythymine sequence was added, and the case where the target nucleic acid of polyadenine sequence was additionally added are indicated. Here, the upper halves are probe regions having polyadenine sequences and the lower halves are probe regions having polythymine sequences.

Fig. 6 is photographs showing the images of degree of polarization taken using the solid phase system according to the invention (mercury lamp excitation). From the left, the case where no target nucleic acid was present, the case where the target nucleic acid of polyadenine sequence was added, and the case where the target nucleic acid of polythymine sequence was additionally added are indicated. Here, the upper halves are probe regions having polyadenine sequence and the lower halves are probe regions having polythymine sequences.

Fig. 7 is a plot showing the dose reaction curve of a target nucleic acid using the solid phase system according to the invention.

Fig. 8 shows the time-dependent variation in the degree of polarization of the solid phase system for detection according to the invention after reaction with a target nucleic acid.

Fig. 9A shows a normal fluorescence image of the solid phase system for detection comprising three regions obtained in Example 14. Here, the upper half of the image indicates a region to which probe DT20BF was bound; the lower right half, a region to which probe TA03BF was bound; and the lower left half, a region to which probe TA05BF was bound.

Fig. 9B shows an image of the degree of polarization of the solid phase system for detection comprising three regions obtained in Example 14 (prior to reaction with a target nucleic acid). Here, the upper half of the image indicates a region to which probe DT20BF was bound; the lower right half, a region to which probe TA03BF was bound; and the lower left half, a region to which probe TA05BF was bound.

Fig. 9C shows an image of the degree of polarization of the solid phase system for detection comprising three regions obtained in Example 14 (after reaction with the target nucleic acid, one kind). Here, the upper half of the image indicates a region to which probe DT20BF was bound; the lower right half, a region to which probe TA03BF was bound; and the lower left half, a region to which probe TA05BF was bound.

Fig. 10A shows a normal fluorescence image of the solid phase system for detection comprising three regions obtained in Example 15. Here, the upper half of the image indicates a region to which probe DT20BF was bound; the lower right half, a region to which probe TA05BF was bound; and the lower left half, a region to which probe TA03BF was bound.

Fig. 10B shows an image of the degree of polarization of the solid phase system for detection comprising three regions obtained in Example 15 (prior to reaction with the target nucleic acid). Here, the upper half of the image indicates a region to which probe DT20BF was bound; the lower right half, a region to which probe TA05BF was bound; and the lower left half, a region to which probe TA03BF was bound.

Fig. 10C shows an image of the degree of polarization of the solid phase system for detection comprising three regions obtained in Example 15 (after reaction with the target nucleic acids, two kinds at the same time). Here, the upper half of the image indicates a region to which probe DT20BF was bound; the lower right half, a region to which probe TA05BF was bound; and the lower left half, a region to which probe TA03BF was bound.

Fig. 11 is a plot of increments in the degree of polarization against the concentrations of an added target nucleic acid when the target nucleic acid was allowed to react with two solid phase systems for detection differing in the density of the immobilized probe, as obtained in Example 19.

Fig. 12 is a plot of increments in the degree of polarization against the concentrations of an added target nucleic acid when the target nucleic acid was allowed to react with the solid phase system for detection to which a probe together with another biotinylated nucleic acid, which would not undergo cross reaction with the probe, were bound, as obtained in Example 20.

**BEST MODE FOR CARRYING OUT THE INVENTION**

As has been explained, the conventional methods for detecting a target nucleic acid based on variation in the degree of polarization of fluorescence have drawbacks. In view of these, the present inventors made thorough investigations and succeeded in discovering a solid phase system for the detection of a nucleic acid, which has a novel structure as well as succeeded in developing a method for the detection, the quantification, or the detection of two-dimensional distribution of a target nucleic acid using such solid phase system; and they have accomplished this invention.

Specifically, this invention provides a solid phase system for the detection of a target nucleic acid and a method for detecting a target nucleic acid, which will be shown below (Nos. 1-7).

1. A solid phase system for the detection of a nucleic acid comprising:

> a probe comprising an oligonucleotide one end of which is provided with a fluorescent dye via a linker and the other end of which is provided with a solid phase bonding part; and
> a solid phase being bound to the probe via the solid phase bonding part.

Specifically, the solid phase system for the detection of a nucleic acid according to this invention is characterized in that it has a structure where the solid phase, the oligonucleotide, and the fluorescent dye are all linked. There are no particular limitations to the bonding positions between the oligonucleotide and the solid phase and between the oligonucleotide and the fluorescent dye.

2. A solid phase system for the detection of a nucleic acid comprising:

> a solid phase having N regions D1-DN on a surface thereof; and
> probe Ci comprising oligonucleotide Bi having base sequence Ai,
> wherein one end of oligonucleotide Bi is provided with a fluorescent dye via a linker and the other end of oligonucleotide Bi is provided with a solid phase bonding part,
> wherein probe Ci is correspondingly bound to region Di via the solid phase bonding part,
> further wherein "N" is an integer of two or more and "i" is an integer of from 1 to N.

Specifically, there are no particular limitations to the size and shape of region Di of the solid phase system for the detection of a nucleic acid according to the invention. Also, "N" is not particularly limited. Further, there may or may not be a partition between regions Di.

3. A solid phase system for the detection of a nucleic acid comprising:

> a solid phase having N regions D1-DN on a surface thereof; and
> a probe comprising an oligonucleotide one end of which is provided with a fluorescent dye via a linker and the other end of which is provided with a solid phase bonding part,
> wherein Pi number of the probe is correspondingly bound to region Di via the solid phase bonding part,
> further wherein "N" is an integer of two or more and "i" is an integer of from 1 to N.

4. A method for detecting a target nucleic acid comprising the steps of:

> (1) adding the target nucleic acid to the surface of a solid phase system for the detection of a nucleic acid comprising:
>
> > a probe comprising an oligonucleotide one end of which is provided with a fluorescent dye via a linker and the other end of which is provided with a solid phase bonding part; and
> > a solid phase being bound to the probe via the solid phase bonding part,
> > to form a hybrid between the target nucleic acid and the probe; and
>
> (2) determining the degree of polarization of fluorescence emitted by the probe.

5. A method for detecting a target nucleic acid comprising the steps of:

> (1) adding the target nucleic acid to the surface of a solid phase system for the detection of a nucleic acid comprising:

a solid phase having N regions D1-DN on a surface thereof; and
probe Ci comprising oligonucleotide Bi having base sequence Ai,
wherein one end of oligonucleotide Bi is provided with a fluorescent dye via a linker and the other end of oligonucleotide Bi is provided with a solid phase bonding part,
wherein probe Ci is correspondingly bound to region Di via the solid phase bonding part,
further wherein "N" is an integer of two or more and "i" is an integer of from 1 to N, and

(2) determining the degree of polarization of fluorescence emitted by the probe.

6. A method for quantifying a target nucleic acid comprising the steps of:

(1) adding the target nucleic acid to the surface of a solid phase system for the detection of a nucleic acid comprising:

a solid phase having N regions D1-DN on a surface thereof; and
a probe comprising an oligonucleotide one end of which is provided with a fluorescent dye via a linker and the other end of whiche is provided with a solid phase bonding part,
wherein Pi number of the probe is correspondingly bound to region Di via the solid phase bonding part,
further wherein "N" is an integer of two or more and "i" is an integer of from 1 to N,
to form a hybrid between the target nucleic acid and the probe; and

(2) determining the degree of polarization of fluorescence emitted by the probe.

7. A method for detecting the two-dimensional distribution of a target nucleic acid comprising the steps of:

(1) trancribing the target nucleic acid onto the surface of a solid phase system for the detection of a nucleic acid comprising:

a probe comprising an oligonucleotide one end of which is provided with a fluorescent dye via a linker and the other end of which is provided via a solid phase bonding part; and
a solid phase being bound to the probe via the solid phase bonding part;

(2) forming a hybrid between the target nucleic acid and the probe; and
(3) determining in a two-dimensional manner, the degree of polarization of fluorescence emitted by the probe.

This invention will be explained in more detail by referring to embodiments hereinbelow.
In the present specification, the following abbreviations will be used, where necessary.

ABBREVIATIONS

DNA    Deoxyribonucleic acid
RNA    Ribonucleic acid
A    Adenine
C    Cytosine
G    Guanine
T    Tymine

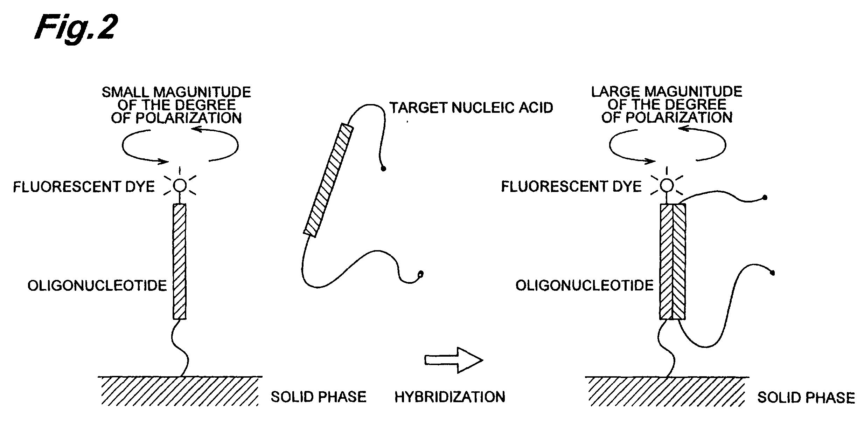

As Figs. A-F schematically show some usable embodiments, the solid phase system for the detection of a nucleic acid according to this invention has a constitution such that it is a probe linked to the surface of a solid phase via a solid phase bonding part, the probe has an oligonucleotide comprising a base sequence capable of hybridizing to a target nucleic acid, and a fluorescent dye is bound to part of the oligonucleotide via a linker.
As Fig. 2 schematically shows, the method for detecting a target nucleic acid according to the invention employs the solid phase system for the detection of a nucleic acid, whereby the base sequence of the probe on the solid phase and part of the target nucleic acid are hybridized to form a hybrid. Consequently, there will be an increase in the degree of polarization of the fluorescence by the fluorescent dye bound to the probe. The presence of the target nucleic acid is to be detected by observing such variation in the degree of polarization.

Target Nucleic Acids

The target nucleic acids that can be detected by the solid phase system for the detection of a nucleic acid according to this invention are not particularly limited, and may be such that they contain known specified base sequences within parts thereof. Therefore, they do not necessarily need to be only comprised of nucleic acid bases and may be those which contain other structures within parts thereof. For example, they embrace those in which sugar, amino acid, and protein components are further bound to nucleic acids. In addition, the target nucleic acids are not only necessarily limited to native DNAs and RNAs, but also encompass synthesized DNAs and RNAs. Furthermore, if the molecular weight of the target nucleic acid is large, it will be made into fragments of preferable base numbers by suitable cleaving means (chemical digestion and cleavage with enzyme), and they may be used in detection.

Solid Phases

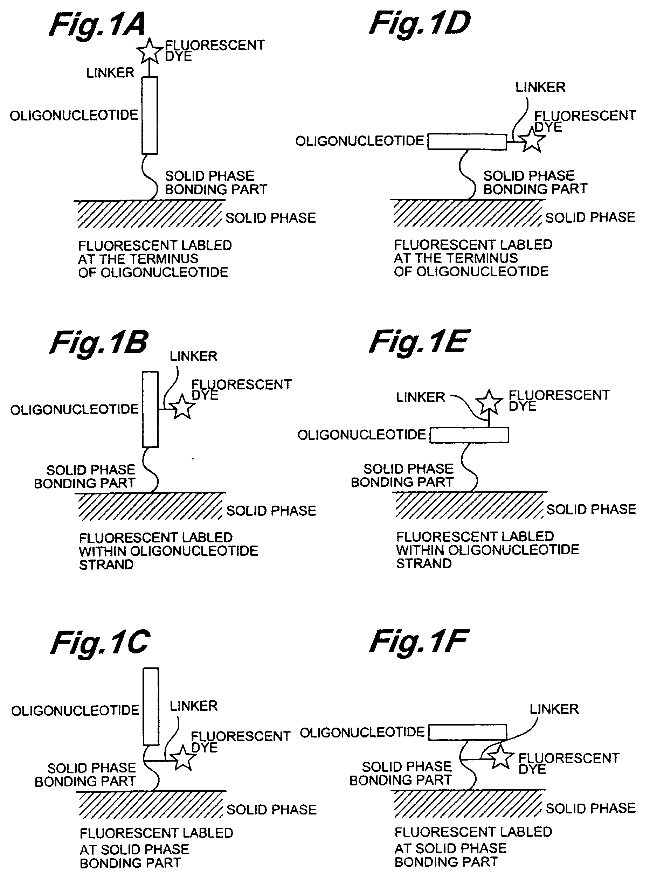

The solid phases that can be used in the solid phase system for the detection of a nucleic acid according to this invention are not particularly limited insofar as they support probes in linking manners, as several embodiments of them are shown in Fig. 1. Materials for such solid phases may be such that they have suitable bonding groups on their surfaces and are stable to a variety of conditions for use in ordinary hybridization. Specifically, mentioned are glasses (plate, film, and particulate forms), metals (plate, film, and particulate forms), and polymer substances (plate, film, and particulate forms).

When a glass is to be used, it is easy to introduce to its surface, various activating groups through oxidation treatment or the like. For the activating group, specifically mentioned are a hydroxyl group, a carboxyl group, and an amino group. For the method to introduce such an activating group, a treatment technique to introduce activating groups into glass surfaces known in the art can be employed.

There is no particular limitation concerning the method for linking a probe (which will be explained below) to the surface of the solid phase, which method relays on the aforementioned activating group on the surface of the solid phase; and a variety of bonding is available for use. Specifically mentioned are a covalent bond, an ionic bond, and a strong interaction between specified proteins. Especially, an antibody-antigen interaction and an avidin-biotin interaction can preferably be used.

Probes

The probe according to this invention that is supported on the surface of the solid phase described above has such a structure that it bears an oligonucleotide comprising a base sequence capable of hybridizing to the target nucleic acid a part of which oligonucleotide is provided with a fluorescent dye via a linker and with a solid phase bonding part and that it is linked to the surface of the solid phase via the solid phase bonding part.

Further, the bonding positions of the fluorescent dye and the solid phase bonding part are not particularly limited. Specifically, as Fig. 1 shows, (I) the position to be linked to the solid phase may be either at one end of the oligonucleotide (Figs. 1A-1C) or at its middle part (Figs. 1D-1F); and (II) the position to which the fluorescent dye is linked may be at one end of the oligonucleotide (Figs. 1A and 1D), at its middle part (Figs. 1B and 1E), or at the solid phase bonding part (Figs. 1C and 1F).

The base sequence of the oligonucleotide describe above is not particularly limited insofar as it is one complementary to the whole or part of the base sequence contained in the nucleic acid to be detected and it is one that specifically binds to the nucleic acid to be detected.

The base number of the oligonucleotide is not particularly limited insofar as it specifically recognize the nucleic acid to be detected and forms a hybrid that is sufficiently stable under standard conditions of measurement. In consideration of the specific recognition of the nucleic acid and the stability of the hybrid, the base number is preferably at least 10 and is more preferably 15 or more. Further, if the base number grows too large, the synthesis of the oligonucleotide will be difficult, for which reason the base number is preferably 100 or less.

Moreover, the linker is able to adjust the distance between the oligonucleotide portion of the probe and the fluorescent dye. The molecular motion of the fluorescent dye is restricted because of its hybridization to the target nucleic acid. The result is that the degree of polarization of fluorescence of said dye varies significantly. Restriction of the molecular motion grows large, as the linker becomes shorter. However, if the linker is too short, the aforementioned hybridization is then restricted by steric hindrance of said dye. As a result, a sufficiently stable hybrid will not be formed. Selection thus may be made as to the length of the linker so that it would not be steric hindrance to the formation of a normal nucleic acid double strand and said formation of a double strand can sufficiently restrict the molecular motion of the fluorescent dye. Such selection can be readily optimized by preparing linkers of different lengths and investigating the correlation between their length and the variation in the degree of polarization of the resultant fluorescence. Specifically, it is preferred that when the length of the linker, i.e., from the terminal of the oligonucleotide to the fluorescent dye, is converted as a methylene chain ($-CH_2)_m-$), "m" be from 2 to 12; m of from 3 to 6 is more preferred.

Furthermore, the structure of the linker is not particularly limited and may be such that it is sufficiently stable under the conditions which are used in the method of this invention. With respect to the structure capable of systematically altering such length of the linker, concrete examples are a methylene chain, an oligonucleotide, an oligopeptide, an oligosaccharide, and a combination of the foregoing, any of which can may be used. The bonding group between the linker and the oligonucleotide is not particularly limited and bonding groups that are ordinarily known in the art can readily be selected. Specifically, a hydroxyl group of an oligonucleotide or a derivative thereof is usable. Individually mentioned are an ester bond group resulting from said hydroxyl and a carboxyl group, a carbamate bond group resulting from said hydroxyl and an isocyanate group, a thiocarbamate bond group resulting from said hydroxyl group and an isothiocyanate group, and a thiourea bond group resulting from an amino-modified oligonucleotide and an isothiocyanate group. Furthermore, the bonding group between the linker and the fluorescent dye is not particularly limited and it can readily be selected, where appropriate, based on a variety of bonding groups which are available from the fluorescent dye molecule. Particularly, when the fluorescent dye bears an isothocyanate group, it is preferred that the bonding group be a thiourea bond group with the linker bearing an amino group or a thiocarbamate bond group with the linker bearing a hydroxyl group.

Moreover, the fluorescent dyes that can be used in this invention are not particularly limited. The larger their variations in the degree of polarization of fluorescence owing to restriction of the molecular motion of the dye, more favorable they are. Specifically mentioned are FITC, BODIPY-FL, acridine, Cy-3, Rhodamine 6G, TRITC, XRITC, FluorX, Rhodamine Green, Rhodol Green, Oregon Green, TAMRA, and ROX. Especially preferred for use are FITC, Rhodol Green, Rhodamine Green, Rhodamine 6G, and TAMRA.

The probe explained above is linked onto the surface of the solid phase via the solid phase bonding part, and its linking means is not particularly limited. As has been explained above, it is sufficient that the probe be provided with a group capable of bonding with various activating groups present on the surface of the solid phase in the vicinity of the other end of its oligonucleotide portion. Bonding based on a variety of known interactions in the art can also be employed. Specifically mentioned are an interaction between proteins and an interaction between nucleic acids. As the interaction between proteins, mentioned are an interaction between avidin and biotin and an interaction between antigen and antibody. The solid phase bonding part needs to maintain a sufficient distance from the surface of the solid phase so that it can adequately hybridize to the target nucleic acid and can form a stable hybrid. The solid phase bonding part does not necessarily need to bind to the terminal of the probe and may bind to it at an appropriate position. If the target nucleic acid is extremely large, the probe is preferably such structure that will enable to change its position in a relatively free manner.

Shape of the Solid Phase

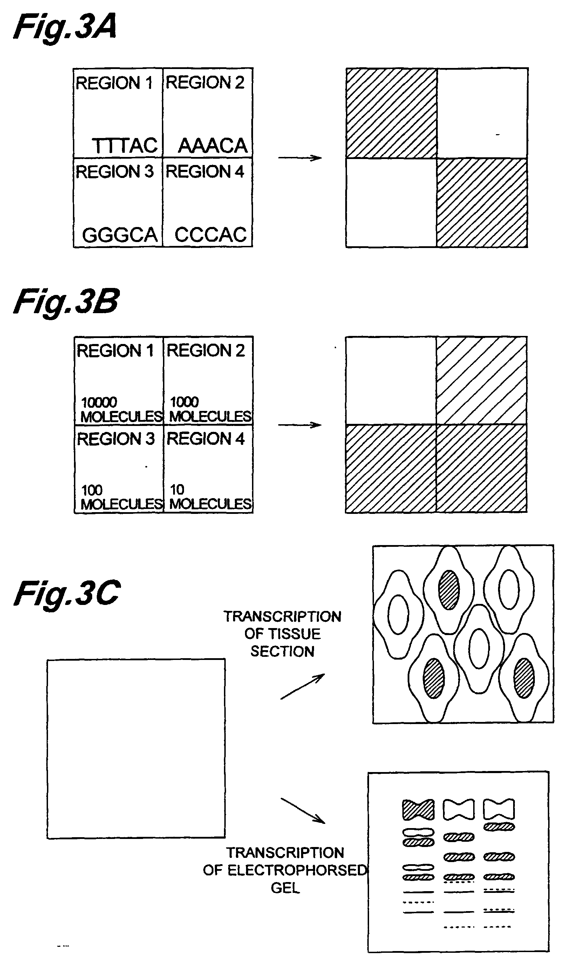

With respect to the structure of the solid phase according to this invention as explained above, it is not limited to one where one kind of probe is bound to the entire surface of the solid phase. Namely, when the surface of the solid phase is divided into plural regions, it is possible to bind each one kind of probe to each region. Figs. 3A-3C show some practical examples.

The embodiment shown in Fig. 3A is as follows: it is prepared by dividing the surface of the solid phase into plural regions (Regions 1-4 in Fig. 3A) and by binding probes that have base sequences different from each other to the respective regions. Such preparation of the solid phase having plural regions is possible by carrying out the preparation method of a solid phase as explained above in each region. Specifically, after the solid phase has been divided into plural regions beforehand, the respective regions are to be shielded by suitable means. Thereafter, the probes are allowed to bind onto the surfaces of unshielded regions, and then, those other than the different regions are shielded; and the probes are allowed to bind onto their surfaces in like manner. By conducting similar treatment in all the regions, there can be obtained a solid phase to which the probes having base sequences differing with respect to individual regions are bound. Alternatively, solid phases to which probes having different base sequences have been bound are linked in advance. It is then possible to obtain a solid phase to which the probes having base sequences differing with respect to individual regions are bound. The resultant solid phase, to which the probes having base sequences differing with respect to individual regions are bound, will enable simultaneous detection of plural target nucleic acids when a mixture comprising the plural target nucleic acids is taken as a sample, which will be explained below.

The embodiment shown in Fig. 3B is as follows: it is prepared by dividing the surface of a solid phase into plural regions and by binding probes that have the same base sequences to the respective regions with differing densities on the solid phase. The resultant solid phase, to which the probes differing in their densities with respect to individual regions are bound, will enable determination of the concentration of the target nucleic acid in a sample, which will be explained below.

The embodiment shown in Fig. 3C is one in which the probe having a specified base sequence is uniformly bound to the entire surface of a solid phase. As will be explained below, such a solid phase will be usable in the histochemical method of a target nucleic acid. In other words, it will be possible to obtain the two-dimensional distribution of a specified target nucleic acid in tissue.

<u>Method for the Detection of a Target Nucleic Acid</u>

The method for detecting a target nucleic acid employing the solid phase, according to this invention, may be carried out by using the solid phase system for the detection of a nucleic acid according to the invention as described above and by determining the degree of polarization of the fluorescence emitted by the fluorescent dye of the probe bound to said solid phase. Namely, the method detects the target nucleic acid through the variation in the degree of polarization resulting from the formation of a hybrid between the probe and the target nucleic acid.

Specifically, the fluorescent dye on the surface of the solid phase is excited by linearly polarized light. The intensity of the polarization component of fluorescence "Ip" that is parallel to the orientation of polarization and the intensity of the polarization component of fluorescence "Is" that is perpendicular to the orientation of polarization are measured; and the degree of polarization "p" or the anisotropy ratio "r" is computed according to the following equations. In the following equations, the constant "G" is the polarization response correction factor of a device.

$$p = (Ip - G \cdot Is)/(Ip + G \cdot Is)$$

$$r = (Ip - G \cdot Is)/(Ip + 2G \cdot Is)$$

Here, the excitation light source is not particularly limited. For example, an ultra high-pressure mercury lamp, an argon laser, a YAG laser, a mode-locked titanium sapphire laser, and the like can preferably be used. Also, the detector is not particularly limited, and a standard point detector (specifically, a photomultiplier) or a two-dimensional detector (specifically, a CCD camera equipped with an image intensifier) is usable. If the two-dimensional detector is used, it will be possible to measure the whole solid phase and thereby to simultaneously observe the respective regions of the solid phase having plural regions. This will make it possible to easily obtain the two-dimensional distribution of the target nucleic acid in a histochemical method. Furthermore, if necessary, a magnification means having a suitable optical system can also be placed for use in combination between the solid phase and the detector.

<u>Method for the Quantification of a Target Nucleic Acid</u>

Utilization of the method according to this invention enables the determination of the concentration of a target nucleic acid in a sample. To this end, a solid phase in the form of the embodiment shown in Fig. 3B can be used, for example. Specifically, the surface of the solid phase is divided into plural regions (four regions 1-4 in Fig. 3B) and a probe having the same base sequence is allowed to bind to each region with the density on the solid phase which has been systematically varied: in the figure, 10,000, 1,000, 100, and 10 molecules, respectively in regions 1, 2, 3, and 4. Further, the sample is added to all the regions on the solid phase, and the degrees of polarization of fluorescence in the respective regions are determined. This will allow the concentration of the target nucleic acid in the sample to be estimated easily and quickly. The figure shows the following case: a sufficient variation is noted in Regions 3 and 4 in which the bonding density between the probe and the solid phase is low and the target nucleic acid is excessive in quantity relative to the probe; an intermediate variation is noted in Region 2; and almost no variation is noted in Region 1 in which the bonding density between the probe and the solid phase is high and the target nucleic acid is small in quantity relative to the probe.

<u>Method for Detection of the Two-Dimensional Distribution of a Target Nucleic Acid</u>

Utilization of the method according to this invention enables detection of the two-dimensional distribution of a target nucleic acid. To this end, a solid phase in the form of the embodiment shown in Fig. 3C can be used, for example. Specifically, a solid phase is prepared which has a surface that is the same as or larger than that of a sample to be detected for its two-dimensional distribution of the target nucleic acid. This solid phase is allowed to contact with the sample to be detected for its two-dimensional distribution of the target nucleic acid, whereby a hybrid between the target nucleic acid and the probe is formed. There is no particular limitation on the sample to be detected for its two-dimensional distribution of the target nucleic acid insofar as it is capable of being contacted by the solid phase system for the detection of a nucleic acid; and for example, a tissue section, an electrophoresed gel, a nucleic acid transcribed filter, and the like can be used. Further, cultured cells or bacteria can be partially digested on the solid phase system for the detection of a nucleic acid, and then, they can be allowed to contact with the solid phase system for the detection of a nucleic acid while maintaining the two-dimensional distribution of the target nucleic acid. Then, the resultant two-dimensional image of the degree of polarization of the fluorescence emitted by the probe represents the two-dimensional distribution of the target nucleic acid in the sample.

This invention will be explained hereinbelow by way of examples; however, the invention is not to be limited to the examples.

## EXAMPLES

(Example 1) <u>The Degree of Polarization of an Immobilized Nucleic Acid</u>

(Preparation of Solid Phase Systems for Detection)

Oligonucleotides comprising 20-40 bases where biotin and an amino group were introduced to the 5'- and 3'-termini respectively were synthesized on an autosynthesizer for nucleic acid, according to the phosphoroamidite solid phase method, and they were allowed to react with FITC (I type: available from Chemicals Dojin Inc.) to produce modified oligonucleotides, which served as probes.

Here, for the introduction of biotin to the 5'-terminus, a Biotin-amidite (available from Perkin-Elmer Corp.) was used, and for the introduction of the amino group to the 3'-terminus, a 3'-Amine-ON CPG (available from PerSeptive Biosystems) was used.

After the synthesized probes were purified by ion-exchange HPLC (purity of not less than 99%), they were lyophilized and dissolved in TE buffer (10 mM Tris-HCl buffer containing 1 mM ethylenediaminetetraacetic acid, pH 8.0) to give concentrations of 25 μM: these served as probe solutions.

Streptavidin (for biochemical use, available from Wako Pure Chemicals Industries, Ltd.) was diluted with PBS to 100 μg/ml, and 150 μl of the streptavidin was loaded onto a glass face (circular region with a diameter of 10 mm) of a glass bottom culture dish (P35-G-101, available from MatTek Corp.). After streptavidin was adsorbed to the glass face by being left at room temperature for 2 h, streptavidin that had not been adsorbed was washed away with PBS. The PBS was prepared by adding water to 9.6 g of Dalbeco PBS(-) Nissui (available from Nissui Pharmaceutical Co. Ltd.) so as to make up for 1 l.

Next, 120 μl of the aforementioned probe solutions, which had been diluted with PBS to 1 μM, was loaded onto the glass faces to which streptavidin had been adsorbed. They were allowed to stand in a dark place at room temperature for 1 h. After the probes were allowed to bind to the glass faces through the reaction between biotin and streptavidin, unreacted probes were washed away with PBS and these served as solid phase systems for detection.

(Preparation of Target Nucleic Acids)

Oligonucleotides comprising 20 and 40 bases that were complementary to the aforementioned probes were synthesized on an autosynthesizer for nucleic acid, according to the phosphoroamidite solid phase method. After the synthesized oligonucleotides were purified by ion-exchange HPLC (purity of not less than 99%), they were lyophilized and dissolved in TE buffer to give concentrations of 25 μM: these served as target nucleic acids.

(Determination of the Degree of Polarization)

The second harmonics (wavelength: 463 nm; pulse width: 200 femto-seconds, cycle frequency: 100-500 kHz) of a mode-locked titanium sapphire laser (pulse oscillation) was used as an excitation light. This excitation light was passed through a polarizer (Grantailor prizm) and a half-wavelength plate (Fresnel wavelength plate), and was introduced into an inverted microscope. An solid phase system for detection provided on the microscope stage was excited by epi-illumination and the then-obtained fluorescence was observed.

In the observation of fluorescence, a dichroic mirror having a cutoff wavelength of 510 nm, an objective lens for fluorescence observation with 20 magnifications, and a band-pass filter having a transmittance wavelength region of from 520 to 560 nm were used. The fluorescence that had passed the band-pass filter was allowed to pass a rotary polarizer (polarizing plate in a sheet form), and it was received by a CCD camera equipped with an image intensifier.

Excitation was carried out in the orientations of p-polarization and s-polarization relative to the dichroic mirror. There were obtained fluorescence image Ipp of the polarization component (p-polarization component) parallel to p-polarization excitation, fluorescence image Ips of the polarization component (s-polarization component) perpendicular to p-polarization excitation, fluorescence image Iss of the polarization component (s-polarization component) parallel to s-polarization excitation, and fluorescence image Isp of the polarization component (p-polarization component) perpendicular to s-polarization excitation. The polarization response correction factor G and degree of polarization p or the anisotropic ratio "r" were computed from the resultant four images according to the following equations.

$$p=(Ipp-G \cdot Ips)/(Ipp+G \cdot Ips)$$

$$r=(Ipp-G \cdot Ips)/(Ipp+2G \cdot Ips)$$

$$G=[(Ipp \cdot Isp)/(Ips \cdot Iss)]^{1/2}$$

(Reaction between the Solid Phase System for Detection and a Target Nucleic Acid (Hybridization))

Solid phase systems for detection employing a polythymine sequence comprising 20 bases and a polythymine sequence comprising 40 bases, as probes, were respectively prepared. The degrees of polarization of the solid phase systems for detection were determined under PBS immersion prior to reaction with target nucleic acids.
Next, a polyadenine sequence comprising 20 bases and a polyadenine sequence comprising 40 bases, which were sequences complementary to the solid phase systems for detection, were diluted with PBS to 250 nM respectively. Each 100 µl was loaded onto the solid phase systems for detection. After allowing to stand in a dark place at room temperature for 30 mm, the surfaces were washed with PBS. The degrees of polarization of the solid phase systems for detection were determined under PBS immersion after reaction with the target nucleic acids.

| the base number of probe | degree of polarization($\times 10^{-3}$) | | |
|---|---|---|---|
| | prior to reaction with the target nucleic acid | after reaction with the target nucleic acid | difference |
| 20 | 238 | 303 | 65 |
| 40 | 230 | 295 | 65 |
| difference | -8 | -8 | |

The data shown in the table are the mean degrees of polarization computed from the fluorescence images (the cycle frequency of pulse excitation light: 500 kHz; integrated time: 20 sec) in the area of 21,401 pixels (circular region with a diameter of 165 pixels: one pixel corresponds to a region of 1.175x1.175 µm on the sample).
These results show that the degree of polarization of the solid phase system for detection is not much dependent on the base number (or molecular weight) of the probe and that it varies greatly with the reaction with a complementary target nucleic acid, i.e., hybrid formation.

(Example 2) Sequence-Recognizing Ability

Similarly to Example 1, solid-phase systems for detection employing a polythymine sequence comprising 40 bases and a polyadenine sequence comprising 40 bases, as the probes, were respectively prepared. The degrees of polarization of the solid phase systems for detection were determined under PBS immersion prior to reaction with target nucleic acids.
Next, a polyadenine sequence comprising 40 bases and a polythymine sequence comprising 40 bases were, respectively, diluted with PBS to 250 nM. Each 100 µl was loaded onto the respective solid phase systems for detection. After allowing to stand in a dark place at room temperature for 30 min, the surfaces were washed with PBS. The degrees of polarization of the solid phase systems for detection were determined under PBS immersion after reaction with the target nucleic acids.

| probe | target nucleic acid | degree of polarization (x10⁻³) | | |
| --- | --- | --- | --- | --- |
| | | prior to reaction with the target nucleic acid | after reaction with the target nucleic acid | difference |
| polythymine sequence | polyadenine sequence | 205 | 305 | 100 |
| polythymine sequence | polythymine sequence | 214 | 211 | -3 |
| polyadenine sequence | polythymine sequence | 207 | 311 | 104 |
| polyadenine sequence | polyadenine sequence | 208 | 222 | 14 |

The data shown in the table are the mean degrees of polarization computed from the fluorescence images (the cycle frequency of pulse excitation light: 100 kHz; integrated time: 10 sec) in the area of 400 pixels (rectangular region with 20x20 pixels: one pixel corresponds to a region of 1.175x1.175 μm on the sample).

These results show that the degree of polarization of the solid phase system for detection hardly varies when the target nucleic acid, which is not complementary, is allowed to react with it (i.e., between polythymine sequences or between polyadenine sequences), but that it varies greatly in a specific manner only when the target nucleic acid, which is complementary, is allowed to react with it (i.e., between the polythymine sequence and the polyadenine sequence).

(Example 3) The Degree of Polarization of a Two Region Solid Phase System

An open-sided rectangular region (4.5x9 mm) was formed on a cover glass (24x40 mm, No. 1 available from Matsunami Glass Ind., Ltd.) using an adhesive tape: one of 9-mm sides was left open and the opened part was connected to the edge of the cover glass. Streptavidin (25 μl), which had been diluted with PBS to 100 μg/ml, was loaded onto the region thus formed. After streptavidin was adsorbed to the glass face by being left at room temperature for 2 h, streptavidin that had not been adsorbed was washed away with PBS.

Next, 25 μl of the aforementioned probe solution, which had been diluted with PBS to 1 μM, was loaded onto the region to which streptavidin had been adsorbed. It was allowed to stand in a dark place at room temperature for 1 h. After the probe was allowed to bind to the glass face through the reaction between biotin and streptavidin, unreacted probe was washed away with PBS. This method formed a cover glass having a region to which the polythymine sequence comprising 40 bases was bound and a cover glass having a region to which the polyadenine sequence comprising 40 bases was bound. The regions differing from each other were adhered onto a slide glass with the opened parts being in close contact, which resulted in a 9x9-mm solid phase system comprising the two regions for detection.

The two regions of this solid phase system for detection were simultaneously irradiated with excitation light (mode-locked titanium sapphire laser). Similarly to Example 1, under PBS immersion, there were obtained the fluorescence images of the polarization component parallel to, and of the polarization component perpendicular to p-polarization excitation, as well as the fluorescence images of the polarization component parallel to, and of the polarization component perpendicular to s-polarization excitation (the cycle frequency of pulse excitation light: 400 kHz, integrated time: 20 sec). In the resultant four fluorescence images, the area of 1600 pixels (rectangular region with 40x40 pixels: one pixel corresponds to a region of 1.175x1.175 μm on the sample) was selected per region to which each probe was bound. The degrees of polarization of the solid phase system for detection prior to reaction with target nucleic acids were respectively determined from the mean fluorescent intensities of those areas according to the equations of Example 1.

Next, the polyadenine sequence comprising 40 bases and the polythymine sequence comprising 40 bases were diluted with PBS to 250 nm. Each 50 μl was loaded onto the solid phase systems for detection. After allowing to stand in a dark place at room temperature for 30 min, the surfaces were washed with PBS. Similarly to the above, under PBS immersion there were obtained the fluorescence images of the polarization components parallel to p-polarization excitation and the polarization components perpendicular to s-polarization excitation. The degrees of polarization of the solid phase systems for detection were computed from the obtained fluorescence images.

(1) When the polyadenine sequence was used as the target nucleic acid

| region on the solid phase system for detection | degree of polarization(x10^{-3}) | | |
|---|---|---|---|
| | prior to reaction with the target nucleic acid | after reaction with the target nucleic acid | difference |
| probe region having the polythymine sequence | 209 | 290 | 81 |
| probe region having the polyadenine sequence | 172 | 221 | 49 |

(2) When the polythymine sequence was used as the target nucleic acid

| region on the solid phase system for detection | degree of polarization(x10^{-3}) | | |
|---|---|---|---|
| | prior to reaction with the target nucleic acid | after reaction with the target nucleic acid | difference |
| probe region having the polyadenine sequence | 175 | 284 | 109 |
| probe region having the polythymine sequence | 164 | 191 | 27 |

These results show that the variation in the degree of polarization when a target nucleic acid is allowed to react with the solid phase system for detection comprising two regions is large in a specific manner only in the region where the probe complementary to the target nucleic acid was bound.

(Example 4) Images of the Degree of Polarization of the Two Region Solid Phase System

The two regions of the solid phase system prepared in Example 3 were simultaneously irradiated with excitation light (mode-locked titanium sapphire laser). Under PBS immersion, there were obtained the fluorescence image Ipp of the polarization component parallel to p-polarization excitation, the fluorescence image Ips of the polarization component perpendicular to p-polarization excitation, the fluorescence image Iss of the polarization component parallel to s-polarization excitation, and the fluorescence image Isp of the polarization component perpendicular to s-polarization excitation (the cycle frequency of pulse excitation light: 400 kHz; integrated time: 20 sec). Then there was obtained a normal fluorescence image of the two region solid phase system ( Ipp+2G • Ips ) and an image of the degree of polarization that had been computed per image according to the equations of Example 1.

Next, the polythymine sequence comprising 40 bases (50 µl), which had been diluted with PBS to 250 nM, was loaded onto this solid phase system for detection as the target nucleic acid. After allowing to stand in a dark place at room temperature for 30 min, the surface was washed with PBS. After a normal fluorescence image and an image of the degree of polarization were similarly obtained, the polyadenine sequence comprising 40 bases was further allowed to react with the solid phase system in like manner, which similarly resulted in a normal fluorescence image and an image of the degree of polarization.

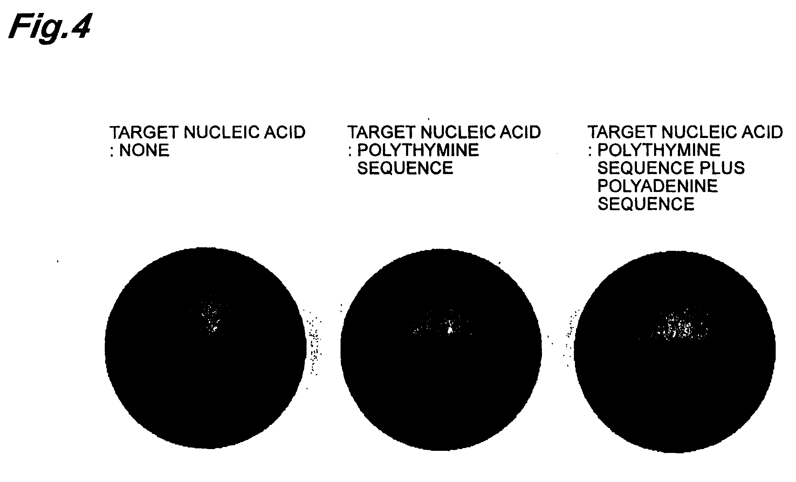

Fig. 4 is photographs showing the normal fluorescence images of the solid phase system for detection comprising two regions. The upper halves of the images show the region to which the probe having the polyadenine sequence was bound; the lower halves of the images show the region to which the probe having the polythymine sequence was bound. As compared to the fluorescence image prior to reaction (left), almost no changes were noted when the polythymine sequence was allowed, as the target nucleic acid, to react with this solid phase system (middle) and when the polyadenine sequence was further allowed to react with the solid phase system (right).

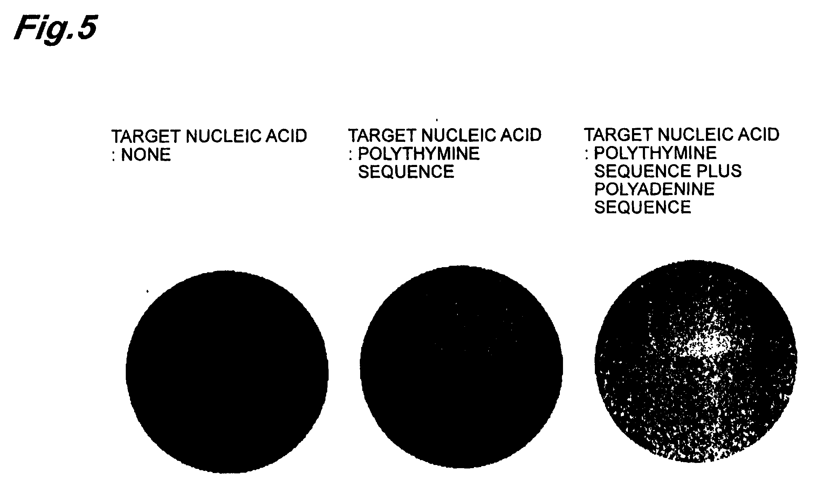

On the other hand, Fig. 5 is photographs showing the images of the degree of polarization for the same regions as in Fig. 4. As compared to the image of the degree of polarization prior to reaction (left), an increase in the degree of polarization was noted only in the region of upper half when the polythymine sequence was allowed, as the target nucleic acid, to react with this solid phase system (middle); an increase in the degree of polarization was noted also in the region of lower half when the polyadenine sequence was further allowed to react with the solid phase system (right).

These results show that the image of degree of polarization allow only the region having formed a hybrid to be differentiated, as being imaged, from other regions.

(Example 5) Determination of the Degree of Polarization of Fluorescence by Excitation with a Mercury Lamp

Using a fluorescence observation device with mercury lamp excitation, the reaction between solid phase systems for detection and target nucleic acids, which was similar to that in Example 1, was observed; and the degrees of polarization of fluorescence were determined.

Specifically, a 100 w mercury lamp was used as an excitation light. This excitation light was passed through a polarizer (polarizing plate in a sheet form), and was introduced into an inverted microscope. The solid phase system for detection provided on the microscope stage was excited by epi-illumination and the then-obtained fluorescence was observed. In the observation of fluorescence, an excitation filter having a transmittance wavelength of from 450 to 490 nm, a dichroic mirror having a cutoff wavelength of 510 nm, an objective lens for fluorescence observation with 20 magnifications, and a band-pass filter having a transmittance wavelength region of from 515 to 565 nm were used. The fluorescence that had passed the band-pass filter was allowed to pass a rotary polarizer (polarizing plate in a sheet form), and it was received by a CCD camera equipped with an image intensifier. Excitation was carried out in the orientations of p-polarization and s-polarization relative to the dichroic mirror. There were obtained the fluorescence image $I_{pp}$ of the polarization component parallel to p-polarization excitation (p-polarization component), the fluorescence image $I_{ps}$ of the polarization component (s-polarization component) perpendicular to p-polarization excitation, the fluorescence image $I_{ss}$ of the polarization component (s-polarization component) parallel to s-polarization excitation, and the fluorescence image $I_{sp}$ of the polarization component (p-polarization component) perpendicular to s-polarization excitation. Similarly to Example 1, the polarization response correction factors G and the degrees of polarization p, or the anisotropic ratios r were computed from the obtained four images.

| the base number of probe | degree of polarization($\times 10^{-3}$) | | |
|---|---|---|---|
| | prior to reaction with the target nucleic acid | after reaction with the target nucleic acid | difference |
| 20 | 130 | 266 | 136 |
| 40 | 128 | 274 | 146 |
| difference | -2 | 8 | |

The data shown in the table are the mean degrees of polarization computed from the fluorescence images in the area of 40,000 or 49,080 pixels (rectangular region of 200x200 pixels or circular region with a diameter of 250 pixels: one pixel corresponds to a region of 1.010x1.010 μm on the sample). The integrated times were 33 sec for the 20 base probe and 10 sec for the 40 base probe.

These results show that the degree of polarization of the solid phase system for detection is not much dependent on the base number (or molecular weight) of probe even if continuous light by mercury lamp excitation is used as the excitation light, but that it varies greatly with reaction with a complementary target nucleic acid, i.e., hybrid formation.

(Example 6) The Degree of Polarization of the Two-Region Solid Phase System by Mercury Lamp Excitation

Reaction with the target nucleic acid was carried out on the solid phase system comprising two regions which was similar to that in Example 3. The regions of the solid phase system for detection were separately irradiated with mercury lamps. Similarly to Example 5, under PBS immersion there were obtained the fluorescence images $I_{pp}$ and $I_{ps}$ of the polarization component parallel to, and of the polarization component perpendicular to p-polarization excitation, as well as the fluorescence images $I_{ss}$ and $I_{sp}$ of the polarization component parallel to, and of the polarization component perpendicular to s-polarization excitation (integrated time: 20 sec). In the resultant four fluorescence images, the areas of 10,000 pixels (rectangular region with 100x100 pixels: one pixel corresponds to a region of 1.010x1.010 μm on the sample) were selected. The degrees of polarization were respectively determined from the mean fluorescent intensities of those areas according to the equations of Example 1.

When the polyadenine sequence was used as the target nucleic acid:

Experiment 1

| region on the solid phase system for detection | degree of polarization($\times 10^{-3}$) | | |
|---|---|---|---|
| | prior to reaction with the target nucleic acid | after reaction with the target nucleic acid | difference |
| probe region having the polythymine sequence | 115 | 264 | 149 |
| probe region having the polyadenine sequence | 105 | 122 | 17 |

Experiment 2

| region on the solid phase system for detection | degree of polarization($\times 10^{-3}$) | | |
|---|---|---|---|
| | prior to reaction with the target nucleic acid | after reaction with the target nucleic acid | difference |
| probe region having the polythymine sequence | 128 | 249 | 121 |
| probe region having the polyadenine sequence | 128 | 108 | -20 |

These results show that also in the case where continuous light by mercury lamp excitation is used as the excitation light, the variation in the degree of polarization is large in a specific manner only in the region to which the probe complementary to a target nucleic acid is bound when the target nucleic acid is allowed to react with the solid phase system for detection comprising two regions.

(Example 7) Images of the Degree of Polarization of the Two Region Solid Phase System by Mercury Lamp Excitation

The two regions of the solid phase system prepared in Example 6 were simultaneously irradiated with excitation light (mercury lamp). Under PBS immersion, there were obtained the fluorescence images of the polarization component parallel to, and of the polarization component perpendicular to p-polarization excitation, as well as the fluorescence images of the polarization component parallel to, and of the polarization component perpendicular to s-polarization excitation (integrated time: 10 sec). Then there was obtained an image of the degree of polarization of the two-region solid phase system that had been computed per image according to the equations of Example 1.

Next, the polyadenine sequence comprising 40 bases (50 μl), which had been diluted with PBS to 250 nM, was loaded onto this solid phase system for detection. After allowing to stand in a dark place at room temperature for 30 min, the surface was washed with PBS. After an image of the degree of polarization was similarly obtained, the polythymine sequence comprising 40 bases was further allowed to react with the solid phase system in like manner, which similarly resulted in an image of the degree of polarization.

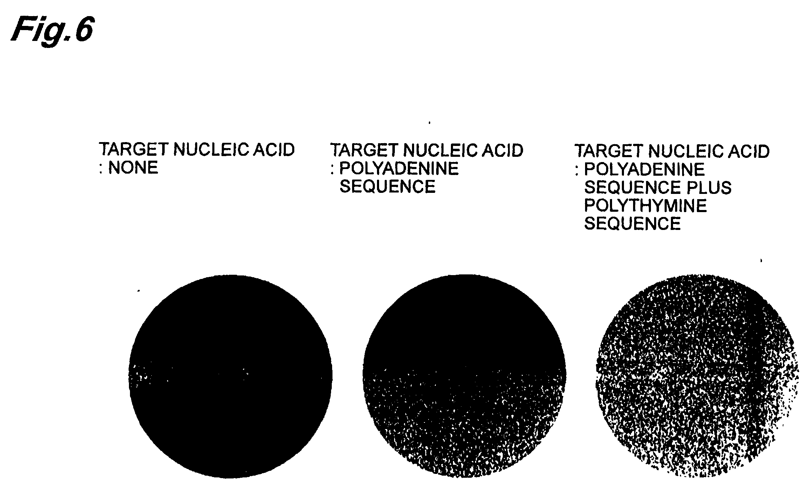

Fig. 6 (left) is diagrams showing the images of degree of polarization of the solid phase system for detection comprising two regions. The upper halves of the images show the region to which the probe having the polyadenine sequence was bound; the lower halves of the images show the region to which the probe having the polythymine sequence was bound. As compared to the image of degree of polarization prior to reaction (a), an increase in the degree of polarization was noted only in the region of lower half when the polyadenine sequence was allowed, as the target nucleic acid, to react with this solid phase system (middle); an increase in the degree of polarization was also noted in the region of upper half when the polythymine sequence was further allowed to react with the solid phase system (right). This indicates that the image of degree of polarization, which have been computed from the fluorescence images by mercury lamp excitation, also allow only the region having formed a hybrid to be differentiated, as being imaged, from other regions.

(Example 8) Dose Reaction Curve

Similarly to Example 1, a solid phase system for detection employing as the probe, the polyadenine sequence comprising 40 bases was prepared.

Next, the polyadenine sequence comprising 40 bases was diluted with PBS to 25 pM-250 nM. Each 100 µl of these or PBS was loaded onto the solid phase systems for detection. After allowing to stand in a dark place at room temperature for 30 min, the surfaces were washed with PBS and the degrees of polarization of the solid phase systems for detection was determined under PBS immersion in a similar manner to Example 4.

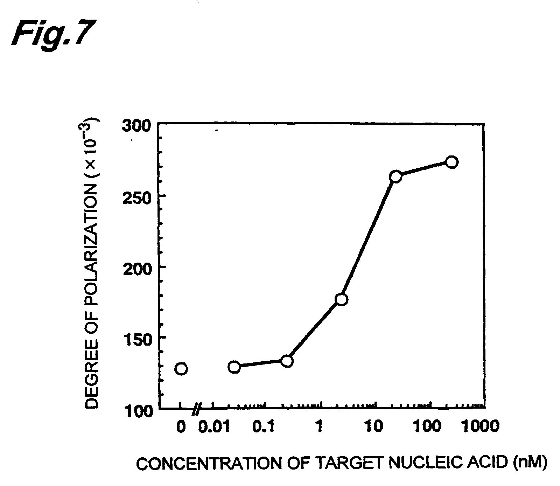

The data shown in Fig. 7 are the mean degrees of polarization computed from the fluorescence images (integrated time: 10 sec) in the area of 40,000 pixels (rectangular region of 200x200 pixels: one pixel corresponds to a region of 1.010x1.010 µm on the sample).

This result shows that the degree of polarization varies depending on the concentration of the target nucleic acid to be reacted with the solid phase system for detection.

(Example 9) Time-Dependent Variation in the Degree of Polarization after Reaction with a Target Nucleic Acid

The probe of the polythymine sequence comprising 40 bases, as shown in Example 1, was prepared; and this was dissolved in TE buffer to give a concentration of 25 µM, which served as the probe solution.

A reaction region of 9x9 mm was formed on a cover glass (24x40 mm, No. 1 available from Matsunami Glass Ind., Ltd.) using silicone rubber in 2-mm thickness. Streptavidin (150 µl), which had been diluted with 1xSSPE to 100 µg/ml, was loaded onto this region. After streptavidin was adsorbed to the glass face by being shaken at room temperature for 2 h, the glass face was washed with 1xSSPE.

Next, 100 µl of the probe solution, which had been diluted with 1xSSPE to 1 µM, was loaded onto the region to which streptavidin had been adsorbed. Through shaking in a dark place at room temperature for 10 min, the probe was allowed to bind to the glass face by reaction between biotin and streptavidin. Thereafter, unreacted probe was washed away with 1xSSPE, which resulted in a solid phase system for detection.

The polyadenine sequence (100 µl) comprising 40 bases, which had been diluted with PBS to 250 nM, was loaded onto this solid phase system for detection as a target nucleic acid. In a time-dependent manner, there were obtained the fluorescence images of the polarization component parallel to, and of the polarization component perpendicular to the excitation of linearly polarized light; and the degrees of polarization were computed from the obtained fluorescence images.

To obtain fluorescence images, a 100 W mercury lamp was used as excitation light. This excitation light was passed through a polarizer (polarizing plate in a sheet form), and was introduced into an inverted microscope (Nikon TE 300, available from Nikon Corp.). The solid phase system for detection provided on the microscope stage was excited by epi-illumination using an objective lens for fluorescence observation with 20 magnifications and a numerical aperture of 0.5 (Nikon Plan Fluor, available from Nikon Corp.). In the observation of fluorescence, an excitation filter having a transmittance wavelength of from 465 to 495 nm, a dichroic mirror having a cutoff wavelength of 505 nm, an objective lens for fluorescence observation with 20 magnifications, and a band-pass filter having a transmittance wavelength region of from 515 to 540 nm were used. The fluorescence that had passed the band-pass filter was separated into two orthogonal polarization components by a Dual View Optical System (available from Hamamatsu Photonics K.K.). The images of the two polarization components were adjusted to be adjacent with each other, and were simultaneously received by one set of CCD camera equipped with an image intensifier.

Excitation was carried out in the orientation of p-polarization relative to the dichroic mirror. Simultaneously, there were obtained the fluorescence images Ipp of the polarization component parallel to p-polarization excitation (p-polarization component), and the fluorescence image Ips of the polarization component (s-polarization component) perpendicular to p-polarization excitation. Excitation was further carried out on the solid phase system for detection, which had been left 10 min after reaction with the target nucleic acid, in the orientation of s-polarization relative to the dichroic mirror. There were then obtained the fluorescence image Iss of the polarization component (s-polarization component) parallel to s-polarization excitation, and the fluorescence image Isp of the polarization component (p-polarization component) perpendicular to s-polarization excitation. The polarization response correction factor G was computed from the resultant four images according to the equations shown in Example 1.

The fluorescence images Ipp and Ips that had been observed at each lapsed time and the polarization response correction factor G that had been derived from the solid phase system for detection left 10 min after reaction with the target nucleic acid were used to compute the degrees of polarization p or the anisotropic ratios r in a similar manner to Example 1.

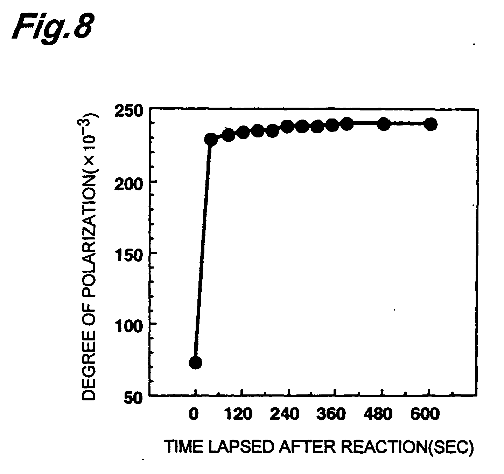

The data shown in Fig. 8 are the mean degrees of polarization computed from the fluorescence images (integrated time: 20 sec) in the area of 73,075 pixels (which corresponds to a rectangular region of 400x200 μm on the sample). The degree of polarization of the solid phase system for detection reached an almost maximum only 40 sec after reaction with the target nucleic acid and then, continued to remain at a high value. This result shows that the solid phase system for detection immediately reacts with a target nucleic acid.

In the present example, the degree of polarization was determined without washing away the target nucleic acid added onto the solid phase system for detection. This indicates that the reaction between the probe on the solid phase and the target nucleic acid can be observed successively by using the same sample without being interrupted.

(Example 10) Variation in the Degrees of Polarization of Probes with Various Fluorescent Dyes

A 40 base oligonucleotide comprising a polythymine sequence where biotin was introduced to the 5'-terminus and an amino group was introduced to the 3'-terminus was prepared according to the method shown in Example 1. Modified oligonucleotides labeled with various fluorescent dyes were produced by reacting this oligonucleotide with the following: Rhodol Green carboxylic acid succinimidyl ester (available from Molecular Probes, Inc.), BODYPY FL C3-succinimidyl ester (available from Molecular Probes, Inc.), Rhodamine Green carboxylic acid succinimidyl ester (available from Molecular Probes, Inc.), Oregon Green carboxylic acid succinimidyl ester (available from Molecular Probes, Inc.), Rhodamine 6G chloride (available from Molecular Probes, Inc.), Fluoro Link Cy3 (available from Amersham Pharmacia Biotech AB), TAMRA-N-hydroxysuccinimide (available from Perkin-Elmer Applied Biosystems), and ROX-N-hydroxysuccinimide (available from Perkin-Elmer Applied Biosystems). These were dissolved in TE buffer to give concentrations of 25 μM, which served as probe solutions.

A reaction region of 9x9 mm was formed on a cover glass (24x40 mm, No. 1 available from Matsunami Glass Ind., Ltd.) using silicone rubber in 2-mm thickness. Streptavidin (150 μl), which had been diluted with 1xSSPE to 100 μg/ml, was loaded onto this region. After streptavidin was adsorbed to the glass face by being shaken at room temperature for 2 h, the glass face was washed with 1xSSPE.

Next, each 100 μl of the probe solutions, which had been diluted with 1xSSPE to 1 μM, was loaded onto the region to which streptavidin had been adsorbed. Through shaking in a dark place at room temperature for 10 min, the probes were allowed to bind to the glass faces by reaction between biotin and streptavidin. Thereafter, unreacted probes were washed away with 1xSSPE, which resulted in solid phase systems for detection.

These solid phase systems for detection were observed under 1xSSPE immersion using the device shown in Example 9. Similarly to example 1, excitation was carried out in the orientations of p-polarization and s-polarization relative to the dichroic mirror. There were obtained the fluorescence image Ipp of the polarization component parallel to p-polarization excitation (p-polarization component), the fluorescence image Ips of the polarization component (s-polarization component) perpendicular to p-polarization excitation, the fluorescence image Iss of the polarization component (s-polarization component) parallel to s-polarization excitation, and the fluorescence image Isp of the polarization component (p-polarization component) perpendicular to s-polarization excitation, respectively. The polarization response correction factors G and the degrees of polarization p or the anisotropic ratios r prior to reaction with a target nucleic acid were computed from the obtained four images.

Next, the polyadenine sequence (100 μl) comprising 40 bases, which had been diluted with 1xSSPE to 250 nM, was loaded onto these solid phase systems for detection as the target nucleic acid. After being shaken in a dark place at room temperature for 10 min, the solid phase systems for detection were replaced by 1xSSPE at their surfaces. These solid phase systems for detection were observed under 1xSSPE immersion using the device shown in Example 9. The polarization response correction factors G and the degrees of polarization p or the anisotropic ratios r after reaction with the target nucleic acid were computed from the obtained four images.

| fluorescent dye | degree of polarization ($\times 10^{-3}$) | | |
|---|---|---|---|
| | prior to reaction with the target nucleic acid | after reaction with the target nucleic acid | difference |
| FITC | 70 | 228 | 158 |
| Rhodol Green | 87 | 242 | 155 |
| BODIPY FL | 70 | 190 | 120 |
| Rhodamine Green | 118 | 283 | 165 |
| Oregon Green | 66 | 162 | 96 |

(continued)

| fluorescent dye | degree of polarization (x10$^{-3}$) | | |
| --- | --- | --- | --- |
| | prior to reaction with the target nucleic acid | after reaction with the target nucleic acid | difference |
| Rhodamine 6G | 138 | 283 | 145 |
| Cy3 | 297 | 342 | 45 |
| TAMRA | 129 | 275 | 146 |
| ROX | 145 | 264 | 119 |

The data shown in the table are the mean degrees of polarization computed from the fluorescence images (integrated time: 20 sec) in the area of 73,075 pixels (which corresponds to a region of 400x200 μm on the sample).

These results show that the degree of polarization significantly rises due to reaction with the target nucleic acid when any of the fluorescent dyes is used. Further, in the cases of FITC, Rhodal Green, Rhodamin Green, Rhodamin 6G, and TAMRA, the variations in the degree of polarization are particularly large; and thus, they are preferable fluorescent dyes in practicing the present method.

(Example 11) Variations in the Degrees of Polarization of Probes with Various Sequences

A β-actin probe comprising 20 bases: 5'-(biotin)-ACCCACACTGTGCCCATCTA-(FITC); and a K-ras probe comprising 20 bases: 5'-(biotin)-GGAGCTGGTGGCGTAGGCAA-(FITC) were prepared according to the method shown in Example 1. These were dissolved in TE buffer to give concentrations of 25 μM, which served as probe solutions.

These probe solutions were used to prepare solid phase systems for detection in a similar manner to Example 10, and they were observed under 1xSSPE immersion using the device shown in Example 9. The degrees of polarization p or the anisotropic ratios r prior to reaction with target nucleic acids were computed in like manner.

Next, a β-actin sequence, 5'-TAGATGGGCACAGTGTGGGT-3', a K-ras sequence, and 5'-TTGCCTACGCCACCAGCTCC-3' were prepared as the target nucleic acids according to the method shown in Example 1. These sequences were diluted with 1xSSPE to 250 nM and the diluted solutions (each 100 μl) were loaded onto the solid phase systems for detection. After being shaken in a dark place at room temperature for 10 min, the solid phase systems for detection were replaced by 1xSSPE at their surfaces. The degrees of polarization p or the anisotropic ratios r after reaction with the target nucleic acids were obtained in like manner.

| probe | degree of polarization (x10$^{-3}$) | | |
| --- | --- | --- | --- |
| | prior to reaction with the target nucleic acid | after reaction with the target nucleic acid | difference |
| 20 base polythymine | 99±3.2 | 243±4.2 | 143 |
| 40 base polythymine | 71±1.5 | 230±2.6 | 159 |
| β-actin probe | 99±0.6 | 225±2.5 | 127 |
| K-ras probe | 164±3.8 | 230±3.5 | 66 |

The data shown in the table represent the mean degrees of polarization and standard deviations obtained when the same experiment was repeated three times. The degree of polarization in each experiment is the mean computed from the fluorescence images (integrated time: 20 sec) in the area of 73,075 pixels (which corresponds to a rectangular region of 400x200 μm on the sample).

These results show that the degree of polarization significantly rises due to reaction with a target nucleic acid also in the solid phase systems to which the β-actin or K-ras probe has been bound.

(Example 12) Variation in the Degree of Polarization with the use of a Linker between the Solid Phase and the Probe

Probes where linkers comprising two and five polyadenine sequences were bound to the 5'-terminus of the β-actin probe shown in Example 11: 5'-(biotin)-AAACCCACACTGTGCCCATCTA-(FITC)-3'; and 5'-(biotin)-AAAAAACCCACACTGTGCCCATCTA-(FITC)-3' were prepared according to the method shown in Example 1. These were dissolved in TE buffer to give concentrations of 25 μM, which served as probe solutions respectively.

These probe solutions were used to prepare solid phase systems for detection in a similar manner to Example 10. They were observed under 1xSSPE immersion using the device shown in Example 9, and the degrees of polarization p or the anisotropic ratios r prior to reaction with a target nucleic acid were obtained in like manner.

Next, the β-actin sequence, which was shown in Example 11 to be a target nucleic acid complementary to these probes, was diluted with 1xSSPE to 250 nM. The diluted solution (100 μl) was loaded onto the solid phase systems for detection. After being shaken in a dark place at room temperature for 10 min, the solid phase systems for detection were replaced by 1xSSPE at their surfaces. The degrees of polarization p or the anisotropic ratios r after reaction with the target nucleic acid were obtained in like manner.

| linker | degree of polarization($\times 10^{-3}$) | | |
| --- | --- | --- | --- |
| | prior to reaction with the target nucleic acid | after reaction with the target nucleic acid | difference |
| none | 99 | 228 | 129 |
| 2 bases | 96 | 209 | 113 |
| 5 bases | 90 | 207 | 117 |

The data shown in the table are the mean degrees of polarization computed from the fluorescence images (integrated time: 20 sec) in the area of 73,075 pixels (which corresponds to a rectangular region of 400x200 μm on the sample).

These results show that the degree of polarization significantly rises due to reaction with a target nucleic acid in the case where the linker comprising a two or five polyadenine sequence is bound to the 5'-terminus of the probe similarly to the case where there is no linker.

(Example 13) Variation in the Degree of Polarization of a Heat-Reactivated Solid Phase System

Similarly to Example 11, a solid phase system for detection to which the K-ras probe comprising 20 bases was bound was prepared. After this solid phase system was washed with 1xSSPE which had been heated at 70 ° C, it was brought back to room temperature and was observed under 1xSSPE immersion using the device shown in Example 9. Thus, the degree of polarization p or the anisotropic ratio r prior to reaction was obtained.

The K-ras sequence (100 μl), which had been diluted with PBS to 250 nM, or only 1xSSPE (100 μl) was loaded onto this solid phase system for detection as the target nucleic acid. After being shaken in a dark place at room temperature for 10 min, the solid phase system for detection was replaced by 1xSSPE at its surface. The degree of polarization p or the anisotropic ratio r after reaction with the target nucleic acid was obtained in like manner.

Next, after this solid phase system was washed again with 1xSSPE which had been heated at 70 ° C, it was brought back to room temperature. The degree of polarization p or the anisotropic ratio r after reactivation was obtained in like manner.

Thereafter, reaction and reactivation was repeated, and degrees of polarization p or anisotropic ratios r were obtained in like manner.

| operation | degree of polarization($\times 10^{-3}$) | |
| --- | --- | --- |
| | reacted with the target nucleic acid | reacted with only 1xSSPE |
| prior to reaction | 197 | 199 |
| reaction (1st time) | 250(53) | 192(-7) |
| reactivation | 204(53) | 207 |

(continued)

| operation | degree of polarization($\times 10^{-3}$) | |
|---|---|---|
| | reacted with the target nucleic acid | reacted with only 1xSSPE |
| reaction (2nd time) | 255(51) | 208(1) |
| reactivation | 214 | 223 |
| reaction (3rd time) | 254(40) | 223(0) |

The data shown in the table are the mean degrees of polarization computed from the fluorescence images (integrated time: 20 sec) in the area of 73,075 pixels (which corresponds to a rectangular region of 400x200 μm on the sample). Values in the parentheses represent increments in the degree of polarization observed when the target nucleic acid or only 1xSSPE was subject to reaction.

The degree of polarization of the solid phase system for detection having reacted with the target nucleic acid proved to be at the same level as that for the solid phase system for detection having reacted with only 1xSSPE when the former solid phase system was washed with 1xSSPE which had been heated at 70 ° C. When this solid phase system was allowed to react with the target nucleic acid, its degree of polarization rose again. This variation in the degree of polarization was also noted even when washing under heating and reaction with the target nucleic acid were repeated.

These results show that the solid phase system having reacted with a target nucleic acid can be reactivated by washing under heating and can repeatedly be used in detecting the target nucleic acid.

(Example 14) <u>Variation in the Degree of Polarization of a Solid Phase System Comprising Three Regions Differing in the Kind of Probe (1)</u>

Probe TA03BF comprising 40 bases: 5'-(biotin)-TTATTTTATATTATTATTATTTATATTTATTTATAATTTT-(FITC)-3'; and TA05BF: 5'-(biotin)-TTTTATTAATTTTTATTTTAATTTATTTTTTAATATTATA-(FITC)-3', both of which would not undergo cross reaction with probe DT20BF of polythymine sequence comprising 20 bases, were prepared similarly to Example 1. These were dissolved in TE buffer to give concentrations of 25 μM, which served as probe solutions respectively.

A reaction zone of 9x9 mm was formed on a cover glass (24x40 mm, No. 1 available from Matsunami Glass Ind., Ltd.) using silicone rubber in 2-mm thickness, and the cover glass was divided into three by being cut with parts in the letter of "T." Streptavidin (50 μl), which had been diluted tenfold with PBS to 100 μg/ml, was loaded onto the respective divided regions. After streptavidin was adsorbed to the glass face by being left at room temperature for 2 h, the glass face was washed with 0.3xSSPE.

Next, each 50 μl of the aforementioned probe solutions, which had been diluted with 0.3xSSPE to 1 μM, was loaded onto the region to which streptavidin had been adsorbed. Through being left in a dark place at room temperature for 10 min, the probes were allowed to bind to the glass faces by reaction between biotin and streptavidin. Thereafter, unreacted probes were washed away with 0.3xSSPE. Subsequently, the three divided regions to which different probes had been bound were pasted together, and thus, the 9x9 mm original reaction zone was regenerated, which resulted in a solid phase system for detection comprising three regions with different kinds of probe.

This solid phase system for detection was observed under 0.3xSSPE immersion with the device shown in Example 9 using an objective lens for fluorescence observation with 10 magnifications and a numerical aperture of 0.3 (Nikon Plan Fluor, available from Nikon Corp.). Then there were obtained the fluorescence images of the polarization component parallel to, and of the polarization component perpendicular to p-polarization excitation, as well as the fluorescence images of the polarization component parallel to, and of the polarization component perpendicular to s-polarization excitation (integrated time: 20 sec). An image of the degree of polarization of the solid phase system comprising three regions prior to reaction with a target nucleic acid was obtained by computation per image element according to the equations of Example 1.

Next, target nucleic acid AT03, which was complementary to TA03BF of these probes, 5'-AAAATTATAAATAAATATAAATAATAATAATATAAAATAA-3' was prepared according to the method shown in Example 1. This target sequence was diluted with 0.3xSSPE to 250 nM and the diluted solution, 100 μl, was loaded on the solid phase system for detection comprising three regions. After allowing to stand in a dark place at room temperature for 10 min, the solid phase system for detection was replaced by 0.3xSSPE at its surface. An image of the degree of polarization after reaction with the target nucleic acid was obtained in like manner.

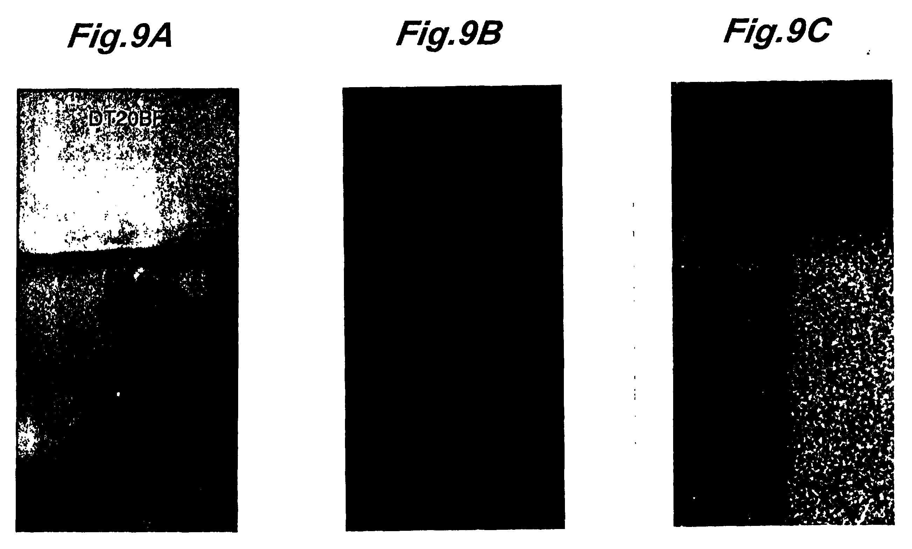

Figs. 9A through 9C are graphs showing the normal fluorescence image (Fig. 9A) of and the images of the degree of polarization of this solid phase system for detection comprising three regions. As is shown in Fig. 9A, the upper half of the image indicates the region to which probe DT20BF was bound; the lower right half, the region to which probe TA03BF was bound; and the lower left half, the region to which TA05BF was bound. As compared to the image of the degree of polarization prior to reaction with the target nucleic acid (Fig. 9B), an increase in the degree of polarization was noted only in the region of lower right half when AT03 was subject to reaction as the target nucleic acid (Fig. 9C).

These results show that the three region solid phase system differing in the kind of probe reacts with a target nucleic acid which corresponds to each of the regions in a specific manner, and that only the degree of polarization in that region rises.

(Example 15) <u>Variation in the Degree of Polarization of the Solid Phase System Comprising Three Regions Differing in the Kind of Probe (2)</u>

Similarly to Example 14, the solid phase system for detection comprising the three regions differing in the kind of probe, which comprised probes DT20BF, TA03BF, and TA05BF, was prepared. An image of the degree of polarization of this solid phase system for detection prior to reaction with a target nucleic acid was obtained by computation per pixel.

A target nucleic acid (DA20) comprising 20 bases, which was complementary to DT20BF of these probes: 5'-AAAAAAAAAAAAAAAAAAAA-3' was prepared according to the method shown in Example 1. 0.3xSSPE (100 μl) was prepared such that it contained each 250 nM of this sequence and target nucleic acid AT03 shown in Example 14, and was loaded onto the solid phase system for detection comprising three regions. After allowing to stand in a dark place at room temperature for 10 min, the solid phase system for detection was replaced by 0.3xSSPE at its surface. An image of the degree of polarization after reaction with the target nucleic acid was obtained in like manner.

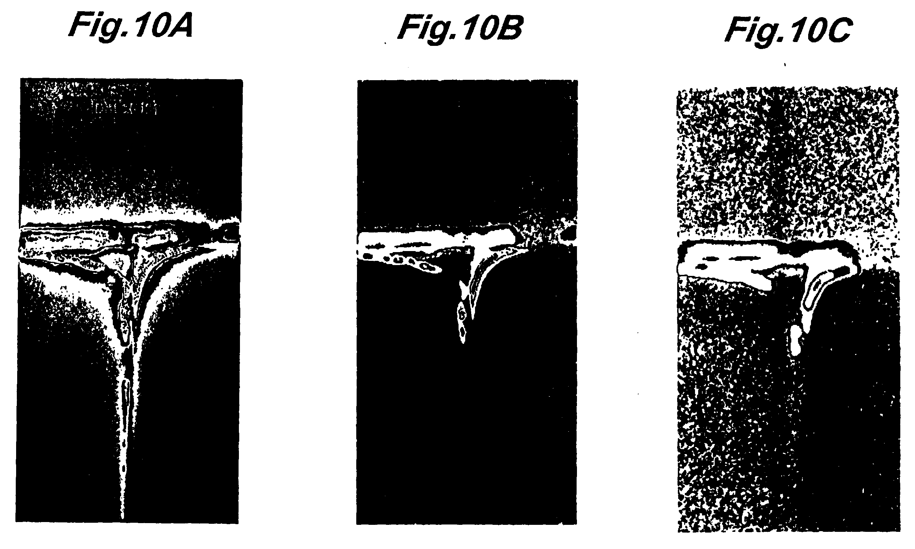

Figs. 10A through 10C are graphs showing the normal fluorescence image (Fig. 10A) of and the image of degree of polarization of this solid phase system for detection comprising three regions (Figs. 10B and 10C). As is shown in Fig. 10A, the upper half of the image indicates the region to which probe DT20BF was bound; the lower right half, the region to which probe TA05BF was bound; and the lower left half, the region to which TA03BF was bound. As compared to the image of the degree of polarization prior to reaction with the target nucleic acid (Fig. 10B), an increase in the degree of polarization was noted both in the region of upper half and in the region of lower left half when a mixture of DA20 and AT03 was simultaneously subject to reaction as the target nucleic acids; whereas, the degree of polarization in the region of lower right half was scarcely varied.

These results show that the three region solid phase system differing in the kind of probe reacts with a target nucleic acid which corresponds to each region in a specific manner even when the two kinds of target nucleic acid are simultaneously allowed to react with the solid phase system for detection, and that only the degree of polarization in that region rises. In other words, the present method is applicable to DNA chips.

(Example 16) <u>Variation in the Degree of Polarization against a Target Nucleic Acid Longer than the Probe Length</u>

The solid phase system to which the β-actin probe comprising 20 bases shown in Example 11 was bound was prepared, and its degree of polarization p or its anisotropic ratio r was obtained under 1xSSPE immersion in like manner.

Next, sequences where three to ten polyadenine sequences were further bound to the 5'-termini of the β-actin sequence shown in Example 11:

5'-AAATAGATGGGCACAGTGTGGGT-3';
5'-AAAAAATAGATGGGCACAGTGTGGGT-3'; and
5'-AAAAAAAAAATAGATGGGCACAGTGTGGGT-3'

were prepared in a similar manner to Example 1. These target sequences were diluted with 1xSSPE to 250 nM and the diluted solution, each 100 μl, was loaded onto the solid phase systems for detection. After being shaken in a dark place at room temperature for 10 min, the solid phase systems for detection were replaced by 1xSSPE at their surfaces. The degrees of polarization p or the anisotropic ratios r after reaction with the target nucleic acids were obtained in like manner.

| the base number of added polyadenine | degree of polarization($\times 10^{-3}$) | | |
|---|---|---|---|
| | prior to reaction with the target nucleic acid | after reaction with the target nucleic acid | difference |
| 10 bases | 101 | 188 | 87 |
| 5 bases | 103 | 173 | 70 |
| 3 bases | 100 | 162 | 62 |
| no addition | 99 | 231 | 132 |

The data shown in the table are the mean degrees of polarization computed from the fluorescence images (integrated time: 20 sec) in the area of 73,075 pixels (which corresponds to a rectangular region of 400x200 µm on the sample).

A significant rise in the degree of polarization was noted when any of the target nucleic acids was subject to reaction, and this result shows that the present method is applicable to a long target nucleic acid longer than that which completely contains a complementary sequence to the probe.

(Example 17) Variation in the Degree of Polarization against a Target Nucleic Acid Shorter than the Probe Length

The solid phase system comprising 25 bases where five polyadenine sequences were bound to the 5'-terminus of the β-actin probe, as shown in Example, 11 was prepared, and its degree of polarization p or its anisotropic ratio r prior to reaction was obtained under 1xSSPE immersion in like manner.

Next, target nucleic acids comprising 20 bases which were complementary to this probe:

TBA20, 5'-TAGATGGGCACAGTGTGGGT-3';
TBA20-1, 5'-AGATGGGCACAGTGTGGGTT-3';
TBA20-3, 5'-ATGGGCACAGTGTGGGTTTT-3'; and
TBA20-5, 5'-GGGCACAGTGTGGGTTTTTT-3'

were prepared in a similar manner to Example 1. These target nucleic acids were diluted with 1xSSPE to 250 nM and the diluted solution, each 100 µl, was loaded onto the solid phase systems for detection. After being shaken in a dark place at room temperature for 10 min, the solid phase systems for detection were replaced by 1xSSPE at their surfaces. The degrees of polarization p or the anisotropic ratios r after reaction with the target nucleic acids were obtained in like manner.

| target nucleic acid | degree of polarization($\times 10^{-3}$) | | |
|---|---|---|---|
| | prior to reaction with the target nucleic acid | after reaction with the target nucleic acid | difference |
| TBA20 | 87 | 205 | 118 |
| TBA20-1 | 88 | 176 | 88 |
| TBA20-3 | 93 | 116 | 23 |
| TBA20-5 | 89 | 73 | -16 |

The data shown in the table are the mean degrees of polarization computed from the fluorescence images (integrated time: 20 sec) in the area of 73,075 pixels (which corresponds to a rectangular region of 400x200 µm on the sample).

When target nucleic acids with 20 bases were allowed to react with the 25 base probe, TBA20 showed a very large rise in its degree of polarization; whereas TBA20-1 showed an intermediate level of rise and almost no variation in the degree of polarization was noted in both TBA20-3 and TBA20-5. The target nucleic acids listed in the upper rows of the table hybridize to the regions close to the 3'-terminus of probe to which a fluorescent dye is bound; therefore, these results show that the formation of double strands in the vicinity of the dye of probe (within three bases) be essential to cause significant variations in the degree of polarization.

(Example 18) <u>Variation in the Degree of Polarization against Target Nucleic Acids Having One Base Mismatch</u>

The solid phase system to which the β-actin probe comprising 20 bases was bound, as shown in Example 11, was prepared, and its degree of polarization p or its anisotropic ratio r prior to reaction was obtained under 1xSSPE immersion in like manner.

Next, target nucleic acids where one base at the 5'-terminus of the β-actin sequence shown in Example 11 was substituted by other bases:

one base-substituted A, 5'-AAGATGGGCACAGTGTGGGT-3';
one base-substituted B, 5'-GAGATGGGCACAGTGTGGGT-3'; and
one base-substituted C, 5'-CAGATGGGCACAGTGTGGGT-3'

were prepared in a similar manner to Example 1. These target nucleic acids were diluted with 1xSSPE to 250 nM and the diluted solution, each 100 μl, was loaded on the solid phase systems for detection. After being shaken in a dark place at room temperature for 10 min, the solid phase systems for detection were replaced by 1xSSPE at their surfaces. The degrees of polarization p or the anisotropic ratios r after reaction with the target nucleic acids were obtained in like manner.

| target nucleic acid | degree of polarization($\times 10^{-3}$) | | |
|---|---|---|---|
| | prior to reaction with the target nucleic acid | after reaction with the target nucleic acid | difference |
| perfect match | 95 | 220 | 125 |
| one base-substituted A | 96 | 151 | 55 |
| one base-substituted A | 96 | 123 | 27 |
| one base-substituted A | 98 | 135 | 37 |

The data shown in the table are the mean degrees of polarization computed from the fluorescence images (integrated time: 20 sec) in the area of 73,075 pixels (which corresponds to a rectangular region of 400x200 μm on the sample).

As compared to the case where the target nucleic acid with a perfect match was subject to reaction, any of the one base substituted target nucleic acids only exhibited a small variation in the degree of polarization. This indicates that the present method is able to recognize only one base difference.

(Example 19) <u>Density of Immobilization and Dose-Reaction Curve</u>

1xSSPEs containing 250 nM and 16 nM of the probe comprising the 40 base polythymine sequence were respectively prepared. Each of these solutions was added to a streptavidin coated 96-well microtiter plate (Black, available from Boehringer Manheim) at 50 μl per well, and it was allowed to stand at room temperature for 30 min. After this plate was washed with 1xSSPE, the target nucleic acid comprising a 40-base polyadenine sequence, which had been serially diluted 4-fold with 1xSSPE to give 1-500 nM, was added to the wells at 100 μl per well, and it was allowed to stand at room temperature for 30 min. The inside of each well was replaced by 100 μl of 1xSSPE, and the degree of polarization of each well at 30 ° C was determined with a fluorescence polarization plate reader (POLARstar, available from BMG Lab Technologies).

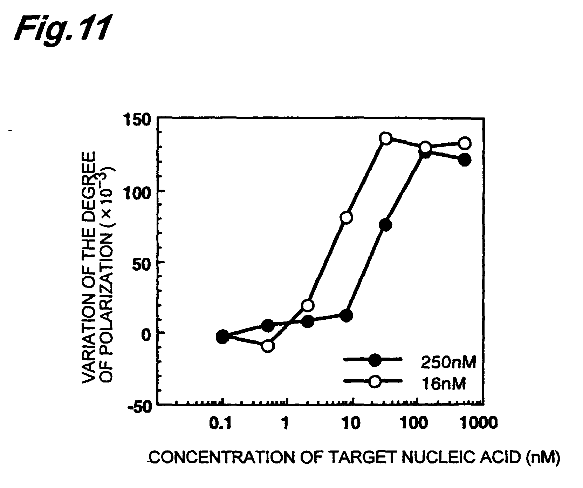

Fig. 11 is a plot of increments in the degree of polarization against the concentrations of the target nucleic acid when the target nucleic acid was subject to reaction. As compared to the wells to which the probe was bound at 250 nM, the graph has shifted to the left when the wells, to which the probe was bound at 16 nM, were used. This indicates that the lower probe density of the solid phase system can allow measurement of a target nucleic acid with higher sensitivity. In other words, it is suggested that in the present method the scope of quantification can be made variable by adjusting the density of probe to be immobilized.

(Example 20) The Ratio of Immobilization and Dose-Reaction Curve

Sequence TA03NF the 5'-terminus of which was biotinylated and which would not undergo cross reaction with the probe comprising a 40 base polythymine sequence:

5'-(biotin)-TTATTTTATATTATTATTATTTATATTTATTTATAATTTT-3'

was prepared similarly to Example 1.

1xSSPE containing 250 nM of the probe comprising a 40 base polythymine sequence and 1xSSPE containing 250 nM of the aforementioned sequence TA03NF were mixed in the volume ratio of 1:3. This solution was added to a streptavidin coated 96-well microtiter plate at 50 μl per well, and a solid phase system for detection was prepared in a similar manner to Example 19. The target nucleic acid comprising the 40 base polyadenine sequence, which had been serially diluted 4-fold with 1xSSPE to give 0.1-500 nM, was added to the wells at 100 μl per well, and they were allowed to stand at room temperature for 30 min. Then, the degree of polarization of each well at 30 ° C was determined with a fluorescence polarization plate reader in like manner.

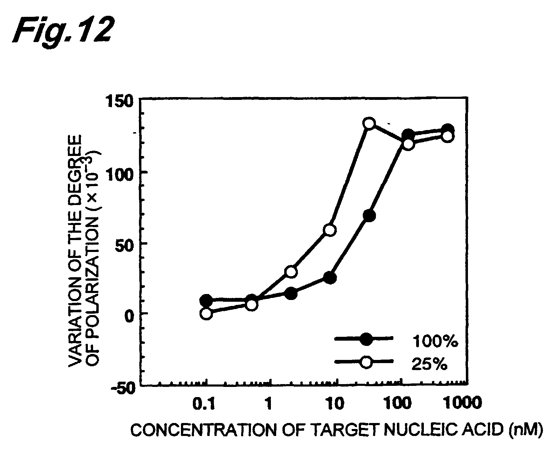

Fig. 12 is a plot of increments in the degree of polarization against the concentrations of the added target nucleic acid when the target nucleic acid was subject to reaction. As compared to the wells (100%) that were prepared using only the 40 base polythymine sequence, the graph has shifted to the left in the case of the wells that were prepared by admixing sequence TA03NF. This indicates that the density of probe of the solid phase system can be adjusted and the scope of quantification can be made variable by admixing other biotinylated target nucleic acids, which do not undergo cross reaction with the probe, at the time of bonding the probe.

**INDUSTRIAL APPLICABILITY**

The solid phase system for the detection of a target nucleic acid according to this invention employs as its basic structure, a structure comprising: a probe comprising an oligonucleotide one end of which is provided with a fluorescent dye via a linker and the other end of which is provided with a solid phase bonding part, wherein the oligonucleotide contains a base sequence complementary to the target nucleic acid; and a solid phase being bound to the probe via the solid phase bonding part. Use of the solid phase system having such a structure enables the formation of a hybrid with the target nucleic acid; and it will be possible to detect the presence of the target nucleic acid by determining the variation in the degree of polarization of the fluorescence resulting from the formation of the hybrid.

Further, the solid phase system, where the surface of a solid phase is divided into plural regions and probes having different base sequences are bound to the respective regions, will enable the simultaneous detection of plural target nucleic acids when a mixture comprising the plural target nucleic acids is taken as a sample.

Furthermore, the solid phase system, where the surface of a solid phase is divided into plural regions, probes having the same base sequence are bound to the respective regions and their concentrations on the solid phase differ, will enable quantification of the concentration of a target nucleic acid in a sample.

Still further, the solid phase system where a probe having a specified base sequence is uniformly bound to the entire surface of a solid phase will make it possible to determine the two-dimensional distribution of a specified target nucleic acid.

**Claims**

1. A solid phase system for the detection of a nucleic acid comprising:

   a probe comprising an oligonucleotide one end of which is provided with a fluorescent dye via a linker and the other end of which is provided with a solid phase bonding part; and
   a solid phase being bound to the probe via the solid phase bonding part.

2. A solid phase system for the detection of a nucleic acid comprising:

   a solid phase having N regions D1-DN on a surface thereof; and
   probe Ci comprising oligonucleotide Bi having base sequence Ai,
   wherein one end of oligonucleotide Bi is provided with a fluorescent dye via a linker and the other end of oligonucleotide Bi is provided with a solid phase bonding part,
   wherein probe Ci is correspondingly bound to region Di via the solid phase bonding part,
   further wherein "N" is an integer of two or more and "i" is an integer of from 1 to N.

3. A solid phase system for the detection of a nucleic acid comprising:

   a solid phase having N regions D1-DN on a surface thereof; and
   a probe comprising an oligonucleotide one end of which is provided with a fluorescent dye via a linker and the other end of which is provided with a solid phase bonding part,
   wherein Pi number of the probe is correspondingly bound to region Di via the solid phase bonding part,
   further wherein "N" is an integer of two or more and "i" is an integer of from 1 to N.

4. A method for detecting a target nucleic acid comprising the steps of:

   (1) adding the target nucleic acid to the surface of a solid phase system for detection of a nucleic acid comprising:

   a probe comprising an oligonucleotide one end of which is provided with a fluorescent dye via a linker and the other end of which is provided with a solid phase bonding part; and
   a solid phase being bound to the probe via the solid phase bonding part,
   to form a hybrid between the target nucleic acid and the probe; and

   (2) determining the degree of polarization of fluorescence emitted by the probe.

5. A method for detecting a target nucleic acid comprising the steps of:

   (1) adding the target nucleic acid to the surface of a solid phase system for detection of a nucleic acid comprising:

   a solid phase having N regions D1-DN on a surface thereof; and
   probe Ci comprising oligonucleotide Bi having base sequence Ai,
   wherein one end of oligonucleotide Bi is provided with a fluorescent dye via a linker and the other end of oligonucleotide Bi is provided with a solid phase bonding part,
   wherein probe Ci is correspondingly bound to region Di via the solid phase bonding part,
   further wherein "N" is an integer of two or more and "i" is an integer of from 1 to N; and

   (2) determining the degree of polarization of fluorescence emitted by the probe.

6. A method for quantifying a target nucleic acid comprising the steps of:

(1) adding the target nucleic acid to the surface of a solid phase system for the detection of a nucleic acid comprising:

a solid phase having N regions D1-DN on a surface thereof; and
a probe comprising an oligonucleotide one end of which is provided with a fluorescent dye via a linker and the other end of which is provided with a solid phase bonding part,
wherein Pi number of the probe is correspondingly bound to region Di via the solid phase bonding part,
further wherein "N" is an integer of two or more and "i" is an integer of from 1 to N,
to form a hybrid between the target nucleic acid and the probe; and

(2) determining the degree of polarization of fluorescence emitted by the probe.

7. A method for detecting the two-dimensional distribution of a target nucleic acid comprising the steps of:

(1) transcribing the target nucleic acid onto the surface of a solid phase for the detection of a nucleic acid comprising:

a probe comprising an oligonucleotide one end of which is provided with a fluorescent dye via a linker and the other end of which is provided with a solid phase bonding part; and
a solid phase being bonded to the probe via the solid phase bonding part;

(2) forming a hybrid between the target nucleic acid and the probe; and
(3) determining in a two-dimensional manner, the degree of polarization of fluorescence emitted by the probe.

## Fig.1A

FLUORESCENT DYE

LINKER

OLIGONUCLEOTIDE

SOLID PHASE BONDING PART

SOLID PHASE

FLUORESCENT LABLED AT THE TERMINUS OF OLIGONUCLEOTIDE

## Fig.1D

LINKER

FLUORESCENT DYE

OLIGONUCLEOTIDE

SOLID PHASE BONDING PART

SOLID PHASE

FLUORESCENT LABLED AT THE TERMINUS OF OLIGONUCLEOTIDE

## Fig.1B

LINKER

FLUORESCENT DYE

OLIGONUCLEOTIDE

SOLID PHASE BONDING PART

SOLID PHASE

FLUORESCENT LABLED WITHIN OLIGONUCLEOTIDE STRAND

## Fig.1E

LINKER

FLUORESCENT DYE

OLIGONUCLEOTIDE

SOLID PHASE BONDING PART

SOLID PHASE

FLUORESCENT LABLED WITHIN OLIGONUCLEOTIDE STRAND

## Fig.1C

OLIGONUCLEOTIDE

LINKER

FLUORESCENT DYE

SOLID PHASE BONDING PART

SOLID PHASE

FLUORESCENT LABLED AT SOLID PHASE BONDING PART

## Fig.1F

OLIGONUCLEOTIDE

LINKER

FLUORESCENT DYE

SOLID PHASE BONDING PART

SOLID PHASE

FLUORESCENT LABLED AT SOLID PHASE BONDING PART

**Fig.2**

SMALL MAGUNITUDE OF THE DEGREE OF POLARIZATION

FLUORESCENT DYE

OLIGONUCLEOTIDE

SOLID PHASE

TARGET NUCLEIC ACID

HYBRIDIZATION

LARGE MAGUNITUDE OF THE DEGREE OF POLARIZATION

FLUORESCENT DYE

OLIGONUCLEOTIDE

SOLID PHASE

## Fig.3A

| REGION 1 | REGION 2 |
|----------|----------|
| TTTAC | AAACA |
| REGION 3 | REGION 4 |
| GGGCA | CCCAC |

→

## Fig.3B

| REGION 1 | REGION 2 |
|----------|----------|
| 10000 MOLECULES | 1000 MOLECULES |
| REGION 3 | REGION 4 |
| 100 MOLECULES | 10 MOLECULES |

→

## Fig.3C

TRANSCRIPTION OF TISSUE SECTION

TRANSCRIPTION OF ELECTROPHORSED GEL

# Fig.4

TARGET NUCLEIC ACID
: NONE

TARGET NUCLEIC ACID
: POLYTHYMINE
SEQUENCE

TARGET NUCLEIC ACID
: POLYTHYMINE
SEQUENCE PLUS
POLYADENINE
SEQUENCE

## *Fig.5*

TARGET NUCLEIC ACID
: NONE

TARGET NUCLEIC ACID
: POLYTHYMINE
  SEQUENCE

TARGET NUCLEIC ACID
: POLYTHYMINE
  SEQUENCE PLUS
  POLYADENINE
  SEQUENCE

EP 1 008 658 A1

# Fig.6

TARGET NUCLEIC ACID
: NONE

TARGET NUCLEIC ACID
: POLYADENINE
  SEQUENCE

TARGET NUCLEIC ACID
: POLYADENINE
  SEQUENCE PLUS
  POLYTHYMINE
  SEQUENCE

EP 1 008 658 A1

## Fig.7

# Fig.8

## Fig.9A

## Fig.9B

## Fig.9C

Fig.10C

Fig.10B

Fig.10A

*Fig.11*

## Fig.12

CONCENTRATION OF TARGET NUCLEIC ACID (nM)

# INTERNATIONAL SEARCH REPORT

| International application No. |
| --- |
| PCT/JP99/00252 |

| A. CLASSIFICATION OF SUBJECT MATTER |
| --- |
| Int.Cl⁶ C12Q1/68, C12N15/10 |

According to International Patent Classification (IPC) or to both national classification and IPC

| B. FIELDS SEARCHED |
| --- |

Minimum documentation searched (classification system followed by classification symbols)
    Int.Cl⁶ C12Q1/68

Documentation searched other than minimum documentation to the extent that such documents are included in the fields searched

Electronic data base consulted during the international search (name of data base and, where practicable, search terms used)
    BIOSIS (DIALOG), WPI (DIALOG)

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y<br>A | JP, 2-75958, A (Kao Corp.),<br>15 March, 1990 (15. 03. 90),<br>Full text ; Fig. 1<br>Full text ; Fig. 1 (Family: none) | 1-5<br>6, 7 |
| Y<br>A | JP, 8-505258, A (R.P. Technologies Inc.),<br>11 June, 1996 (11. 06. 96),<br>Full text ; Figs. 1 to 9<br>Full text ; Figs. 1 to 9<br>& WO, 9412665, A1 & US, 5445935, A<br>& EP, 672191, A1 & US, 5756292, A | 1-5<br>6, 7 |

| [x] Further documents are listed in the continuation of Box C. | [ ] See patent family annex. |
| --- | --- |

| Date of the actual completion of the international search<br>20 April, 1999 (20. 04. 99) | Date of mailing of the international search report<br>27 April, 1999 (27. 04. 99) |
| --- | --- |
| Name and mailing address of the ISA/<br>    Japanese Patent Office | Authorized officer |
| Facsimile No. | Telephone No. |

Form PCT/ISA/210 (second sheet) (July 1992)

## INTERNATIONAL SEARCH REPORT

International application No.

PCT/JP99/00252

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP, 2-295496, A (Imperial Chemical Industries PLC.),<br>6 December, 1990 (06. 12. 90),<br>Full text ; Figs. 1 to 3<br>Full text ; Figs. 1 to 3<br>& EP, 382433, A2 & GB, 2228998, A<br>& AU, 9049025, A & NO, 9000582, A<br>& CA, 2009454, A & FI, 9000602, A<br>& ZA, 9000912, A & ES, 2109226, A<br>& DE, 69031665, A | 1-5<br>6, 7 |
| Y<br>A | JP, 5-236997, A (Hitachi,Ltd.),<br>17 September, 1993 (17. 09. 93),<br>Full text ; Figs. 1 to 6<br>Full text ; Figs. 1 to 6<br>& US, 5434049, A & US, 5607646, A<br>& US, 5817506, A | 1-5<br>6, 7 |
| A | JP, 6-43159, A (Toyobo Co., Ltd.),<br>18 February, 1994 (18. 02. 94),<br>Full text ; Figs. 1 to 4 (Family: none) | 1-7 |
| A | JP, 5-123196, A (Toyobo Co., Ltd.),<br>21 May, 1993 (21. 05. 93),<br>Full text ; Figs. 1 to 4 (Family: none) | 1-7 |

Form PCT/ISA/210 (continuation of second sheet) (July 1992)

SEQUENCE LISTING
<110> Laboratory of Molecular Biophotonics
<120>Solid material for detecting a nucleic acid, and the method
for detecting a nucleic acid
<130> BBP98-04PCT
<160>33


<210> 1
<211>20
<213> artificial sequence
<220>
<223> probe: 5'-biotin; 3'-FITC
<400>1
tttttttttt tttttttttt 20


<210> 2
<211>40
<213> artificial sequence
<220>
<223> probe: 5'-biotin; 3'-FITC
<400>2
<2>40
tttttttttt tttttttttt tttttttttt tttttttttt 40


<210> 3
<211>20
<213> artificial sequence
<220>
<223> target nucleic acid
<400>2
aaaaaaaaaa aaaaaaaaaa 20


<210> 4
<211>40
<213> artificial sequence
<220>
<223> target nucleic acid
<400> 4
aaaaaaaaaa aaaaaaaaaa aaaaaaaaaa aaaaaaaaaa 40


<210> 5
<211>40
<213> artificial sequence
<220>
<223> probe: 5'-biotin; 3'-FITC

```
<400> 5
aaaaaaaaaa aaaaaaaaaa aaaaaaaaaa aaaaaaaaaa 40


<210> 6
<211>40
<213> artificial sequence
<220>
<223> target nucleic acid
<400> 6
tttttttttt tttttttttt tttttttttt tttttttttt 40


<210> 7
<211>40
<213> artificial sequence
<220>
<223> probe: 5'-biotin; 3'-Rhodol Green
<400> 7
tttttttttt tttttttttt tttttttttt tttttttttt 40


<210> 8
<211>40
<213> artificial sequence
<220>
<223> probe: 5'-biotin; 3'-BODIPY FL
<400>8
tttttttttt tttttttttt tttttttttt tttttttttt 40


<210> 9
<211>40
<213> artificial sequence
<220>
<223> probe: 5'-biotin; 3'-Rhodamine Green
<400>19
tttttttttt tttttttttt tttttttttt tttttttttt 40


<210> 10
<211>40
<213> artificial sequence
<220>
<223> probe: 5'-biotin; 3'-Oregon Green
<400>10
tttttttttt tttttttttt tttttttttt tttttttttt 40


<210> 11
<211>40
```

```
<213> artificial sequence
<220>
<223> probe: 5'-biotin; 3'-Rhodamine 6G
<400>11
tttttttttt tttttttttt tttttttttt tttttttttt 40


<210> 12
<211>40
<213> artificial sequence
<220>
<223> probe: 5'-biotin; 3'-Cy3
<400>12
tttttttttt tttttttttt tttttttttt tttttttttt 40


<210> 13
<211>40
<213> artificial sequence
<220>
<223> probe: 5'-biotin; 3'-TAMRA
<400>13
tttttttttt tttttttttt tttttttttt tttttttttt 40


<210> 14
<211>40
<213> artificial sequence
<220>
<223> probe: 5'-biotin; 3'-ROX
<400>14
tttttttttt tttttttttt tttttttttt tttttttttt 40


<210> 15
<211>20
<213> artificial sequence
<220>
<223> probe: 5'-biotin; 3'-FITC: for detection of βactin
<400>15
acccacactg tgcccatcta 20


<210> 16
<211>20
<213> artificial sequence
<220>
<223> probe: 5'-biotin; 3'-FITC: for detection of k-ras
<400>16
ggagctggtg gcgtaggcaa 20
```

<210> 17
<211>20
<213> artificial sequence
<220>
<223> target nucleic acid $\beta$ actin
<400>17
tagatgggca cagtgtgggt 20


<210> 18
<211>20
<213> artificial sequence
<220>
<223> target nucleic acid k-ras
<400>18
ttgcctacgc caccagctcc 20


<210> 19
<211>22
<213> artificial sequence
<220>
<223> probe: 5'-biotin; 3'-FITC: for detection of $\beta$ actin
<400>19
aaacccacac tgtgcccatc ta 22


<210> 20
<211>25
<213> artificial sequence
<220>
<223> probe: 5'-biotin; 3'-FITC: for detection of $\beta$ actin
<400>20
aaaaaaccca cactgtgccc atcta 25


<210> 21
<211>40
<213> artificial sequence
<220>
<223> probe: 5'-biotin; 3'-FITC
<400>21
ttattttata ttattattat ttatatttat ttataatttt 40


<210> 22
<211>40
<213> artificial sequence
<220>

```
<223> probe: 5'-biotin; 3'-FITC
<400>22
ttttattaat ttttatttta atttattttt taatattata 40


<210> 23
<211>40
<213> artificial sequence
<220>
<223> target nucleic acid
<400>23
aaaattataa ataaatataa ataataataa tataaaataa 40


<210> 24
<211>23
<213> artificial sequence
<220>
<223> target nucleic acid: β actin-modified
<400>24
aaatagatgg gcacagtgtg ggt 23


<210> 25
<211>25
<213> artificial sequence
<220>
<223> target nucleic acid: β actin-modified
<400>25
aaaaatagat gggcacagtg tgggt 25


<210> 26
<211>30
<213> artificial sequence
<220>
<223> target nucleic acid: β actin-modified
<400>26
aaaaaaaaaa tagatgggca cagtgtgggt 30


<210> 27
<211>20
<213> artificial sequence
<220>
<223> target nucleic acid: β actin-modified
<400>27
agatgggcac agtgtgggtt 20


<210> 28
```

```
<211>20
<213> artificial sequence
<220>
<223> target nucleic acid: βactin-modified
<400>28
atgggcacag tgtgggtttt 20


<210> 29
<211>20
<213> artificial sequence
<220>
<223> target nucleic acid: βactin-modified
<400>29
gggcacagtg tgggtttttt 20


<210> 30
<211>20
<213> artificial sequence
<220>
<223> target nucleic acid: βactin-modified
<400>30
aagatgggca cagtgtgggt 20


<210> 31
<211>20
<213> artificial sequence
<220>
<223> target nucleic acid: βactin-modified
<400>31
gagatgggca cagtgtgggt 20


<210> 32
<211>20
<213> artificial sequence
<220>
<223> target nucleic acid: βactin-modified
<400>32
cagatgggca cagtgtgggt 20


<210> 33
<211>40
<213> artificial sequence
<220>
<223> probe: 5'-biotin
<400>33
```

ttattttata ttattattat ttatatttat ttataatttt 40

.